(19) Europäisches Patentamt
European Patent Office
Office européen des brevets

(11) Veröffentlichungsnummer: **0 276 805 B1**

# (12) EUROPÄISCHE PATENTSCHRIFT

(45) Veröffentlichungstag der Patentschrift: **17.03.93**

(21) Anmeldenummer: **88101057.3**

(22) Anmeldetag: **26.01.88**

Die Akte enthält technische Angaben, die nach dem Eingang der Anmeldung eingereicht wurden und die nicht in dieser Patentschrift enthalten sind.

(51) Int. Cl.5: **C07D 513/04**, C07D 253/06, A61K 31/53, //(C07D513/04, 277:00,253:00)

## (54) Substituierte 3-Phenyl-7H-thiazolo[3,2 -b] [1,2,4]triazin-7-one, Verfahren zu ihrer Herstellung, die sie enthaltenden Arzneimittel und ihre Verwendung, sowie einige bei der Herstellung der genannten Verbindungen gebildete Zwischenprodukte.

(30) Priorität: **30.01.87 DE 3702758**

(43) Veröffentlichungstag der Anmeldung:
**03.08.88 Patentblatt 88/31**

(45) Bekanntmachung des Hinweises auf die Patenterteilung:
**17.03.93 Patentblatt 93/11**

(84) Benannte Vertragsstaaten:
**AT BE CH DE ES FR GB GR IT LI LU NL SE**

(56) Entgegenhaltungen:
**EP-A- 0 164 765**
**DE-A- 3 042 295**
**DE-A- 3 146 300**

**CHEMICAL ABSTRACTS, Band 92, Nr. 9, 3. März 1980, Seite 676, Zusammenfassung-Nr. 76459b, Columbus, Ohio, US; E.S.A. IBRAHIM et al.: "Potential antineoplastics. Part 2. New thiazolo[1,2,4]triazine derivatives"**

(73) Patentinhaber: **HOECHST AKTIENGESELLSCHAFT**
**Postfach 80 03 20**
**W-6230 Frankturt am Main 80(DE)**

(72) Erfinder: **Thorwart, Werner, Dr.**
**Am Gänsborn 3b**
**W-6203 Hochheim am Main(DE)**
Erfinder: **Gebert, Ulrich, Dr.**
**Albert-Lortzing-Strasse 2**
**W-6233 Kelkheim (Taunus)(DE)**
Erfinder: **Schleyerbach, Rudolf, Dr.**
**Finkenweg 10**
**W-6238 Hofheim am Taunus(DE)**
Erfinder: **Bartlett, Robert R., Dr.**
**Sandbergstrasse 20**
**W-6100 Darmstadt(DE)**

EP 0 276 805 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMISCHE BERICHTE, Band 110, Nr. 4, 1977, Seiten 1492-1496, Verlag Chemie GmbH, Weinheim, DE; G. TOTH et al.: "Zur Ring-Ketten-Tautomerie bei 3-Hydroxythiazolo[3,2-b]-as-triazin-7-onen"

CHEMICAL ABSTRACTS, Band 68, Nr. 7, 12. Februar 1968, Seiten 2883-2884, Zusammenfassung-Nr. 29682p, Columbus, Ohio, US; G. DOLESCHALL et al.: "1,2,4-triazines and condensed derivatives. III. The synthesis of some 7H-thiaziolo[3,2-b]-1,2,4-triazin-7-ones"

JOURNAL OF THE CHEMICAL SOCIETY, PERKIN TRANSACTIONS II, 1980, Seiten 890-899; R.E. BUSBY et al.: "The rearrangement of 2-amino-1,3,4-thiadiazines to 3-amino-2-thiazolimines. Part 1. The rates of rearrangement of a series of 5-alkyl- and 5-aryl-2-amino-1,3,4-thiadiazines at 30 and 50 degrees C"

ZEITSCHRIFT FUER CHEMIE, Band 15, Nr. 12, 1975, Seite 582; E. BULKA et al.: "Ueber die Synthese von 2H-Imidazo[2,1-b]-[1,3,4]-thiadiazinen"

PATENT ABSTRACTS OF JAPAN, Band 8, Nr. 106 (C-223) [1543], 18. Mai 1984; SUMITOMO KAGAKU KOGYO K.K.: "4-allyl-1,2,4-triazin-5(2H)-one-3-thione derivative and its preparation"

CHEMICAL ABSTRACTS, Band 104, Nr. 7, 17. Februar 1986, Seite 513, Zusammenfassung-Nr. 50789a, Columbus, Ohio, US; TEIKOKU HORMONE MFG. CO., LTD.: "3-substituted-2-phenylindole derivatives"

CHEMICAL ABSTRACTS, Band 90, Nr. 15, 09. April 1979, Seite 28, Zusammenfassung-Nr. 114920p, Columbus, Ohio, US; H. VAN DER GOOT et al.: "The synthesis and anti-inflammatory activity of substituted 2-(4-hydroxyphenyl)-1,3-indandiones"

CHEMICAL ABSTRACTS, Band 82, Nr. 9, 03. September 1975, Seite 579, Zusammenfassung-Nr. 57744t, Columbus, Ohio, US; N. YOSHIDA et al.: "2-amino-1,3,4-thiadiazine derivatives"

CHEMICAL ABSTRACTS, Band 85, Nr. 23, 06. Dezember 1978, Seite 521, Zusammenfassung-Nr. 177376h, Columbus, Ohio, US; I.YA. POSTOVSKII et al.: "Synthesis of 2-hydrazino- and 2-amino-1,3,4-thiadiazines containing residues of polyhydric phenols in position 5"

## Beschreibung

Die vorliegende Erfindung betrifft neue mehrfach substituierte 3-Phenyl-7H-thiazolo[3,2-b][1,2,4]triazin-7-one, Verfahren zu ihrer Herstellung und ihre Verwendung als Wirkstoffe in Arzneimitteln insbesondere zur Behandlung rheumatischer Erkrankungen; außerdem bezieht sich die Erfindung auch noch auf einige bei der Herstellung der vorerwähnten Verbindungen gebildete Zwischenprodukte.

Bei den bisher in der Rheumatherapie vorzugsweise eingesetzten nicht-steroidalen Antiphlogistika handelt es sich fast ausschließlich um verhältnismäßig starke Cyclooxygenase-Inhibitoren, die den endogenen Abbau der Arachidonsäure zu den entzündungs- und schmerzfördernden Prostaglandinen hemmen. Allerdings stehen im ursächlichen Zusammenhang mit einer zu starken Hemmung der Cyclooxygenase-Aktivität eine Reihe von gravierenden Nebenwirkungen, wie gastrointestinale Beschwerden, Nierenfunktionsstörungen und allergische Reaktionen (z.B. Hautallergien und Asthmaanfälle), die insbesondere bei der üblicherweise notwendigen Langzeitbehandlung häufig zum Abbruch der Therapie zwingen (vergl. K. Brune, Eur. J. Rheumatol. Inflam. 5, 335-349, 1982).

Ein weiterer mit dem beschriebenen Wirkungsmechanismus ursächlich verbundener Nachteil dieser klassischen nichtsteroidalen Antiphlogistika besteht darin, daß sie zwar die Beseitigung oder Linderung der Symptome Schermz, Entzündung und Schwellung gestatten, aber die den entzündlich rheumatischen Erkrankungen zugrundeliegenden immunpathologischen Prozesse unbeeinflußt lassen und daher den progredienten Krankheitsverlauf nicht aufzuhalten vermögen.

Es besteht somit ein dringendes Bedürfnis an therapeutisch nutzbaren Antirheumatika, die sich aufgrund eines günstigeren Wirkungsprofils von den bekannten nichtsteroidalen Antiphlogistika vorteilhaft durch bessere Verträglichkeit einerseits und einen mehr kausalen Eingriff in das rheumatische Krankheitsgeschehen andererseits unterscheiden. Erfolgversprechende Ansatzpunkte hierfür bieten solche Pharmaka, die verstärkt in den alternativen Weg des Arachidonsäureabbaus eingreifen, indem sie beispielsweise die 5-Lipoxygenase hemmen und somit die exzessive Bildung der proinflammatorischen Leukotriene unterbinden, hochreaktive Sauerstoffradikale, die als Entzündungsmediatoren die Zell- und Gewebszerstörung in den entzündlich rheumatischen Gelenken perpetuell unterhalten, desaktivieren und/oder das entgleiste Immumsystem restaurieren und damit die Möglichkeit eröffnen, mehr ursächlich die rheumatischen Erkrankungen medikamentös zu behandeln.

Überraschend wurde nun gefunden, daß durch Einführung bestimmter 3-substituierter 5-tert.-butyl-4-hydroxyphenyl-Reste in die 3-Stellung von gegebenenfalls in 2- und/oder 6-Position substituierten 7H-Thiazolo[3,2-b][1,2,4]triazin-7-onen neuartige Verbindungen erhalten werden, die aufgrund ihrer pharmakologischen Eigenschaften die zuvor aufgestellten Anforderungen erfüllen und sich demnach hervorragend zur Behandlung rheumatischer Erkrankungen eignen.

Die auch gastral äußerst gut verträglichen Verbindungen hemmen im Unterschied zu den bekannten nichtsteroidalen Antiphlogistika das Arachidonsäure-abbauende Enzym 5-Lipoxygenase, während eine Beeinflussung der Cyclooxygenase nicht nachgewiesen werden kann. Die Fähigkeit der Verbindungen, Sauerstoffradikale zu desaktivieren, zeigt sich beispielsweise im Modell der (R)Adriamycin (Fa. Farmitalia)-induzierten Entzündung und durch die Inhibierung der Lipidperoxydation.

Darüber hinaus greifen sie vorteilhaft in das gestörte Immunsystem ein, wie sich durch Unterdrückung der Arthus-Reaktion und durch Normalisierung der supprimierten Immunaktivität in den pathologischen Modellen der mit Freundschem Adjuvans oder Typ II-Collagen induzierten Arthritis demonstrieren läßt.

Aus der Literatur sind bereits einige 7H-Thiazolo[3,2-b] [1,2,4]triazin-7-one bekannt. So stellten F. Soliman et al. auf der Suche nach antineoplastisch aktiven Verbindungen das 6-(3-Jodstyryl)-3-methyl-7H-thiazolo[3,2-b][1,2,3]tri= azin-5-on her, ohne aber über dessen pharmakologische Eigenschaften zu berichten (Pharmazie 34 (1979), S. 392-394). Dasselbe Ringsystem mit 6-ständigem Alkyl- bzw. Phenylrest wurde von W. Klose et al. (Liebigs Ann. Chem. 1984, S. 1302-1307) aufgebaut; diese Verbindungen sollen herbizide Wirkung besitzen. In der DE-OS 31 46 300 werden schließlich neben einer ganzen Reihe partiell hydrierter 7H-Thiazolo[3,2-b][1,2,4]triazin-7-one das 3,6-Diphenyl-7H-thiazolo[3,2-b][1,2,4]triazin-7-on beschrieben und das entsprechende 3-Methyl-6-phenyl-Derivat erwähnt, die ebenfalls herbizid wirksam sein sollen.

Die vorliegende Erfindung betrifft demgegenüber neue 7H-Thiazolo[3,2-b][1,2,4]triazin-7-one, die in 3-Stellung eine 3-substituierte -5-tert.-butyl-4-hydroxyphenyl-Gruppe und gegebenenfalls in 2- und/oder 6-Position weitere Substituenten tragen, wobei allerdings die Einführung eines Phenylrestes in die 6-Stellung des bicyclischen Systems zu vollständigem Wirkungsverlust führt. Aufgrund ihrer zuvor erwähnten pharmakologischen Eigenschaften eignen sich die erfindungsgemäßen Verbindungen zur Verwendung in Arzneimitteln, insbesondere in solchen, die bei entzündlich rheumatischen Erkrankungen indiziert sind.

Gegenstand der Erfindung sind somit substituierte 3-Phenyl-7H-thiazolo [3,2-b][1,2,4]triazin-7-one der allgemeinen Formel I, in der

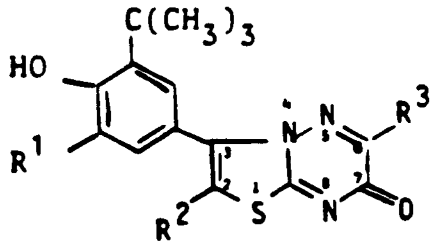

(I)

$R^1$ eine geradkettige oder verzweigte Alkylgruppe mit 1 bis 4 C-Atomen, Hydroxymethyl oder eine Aminomethylgruppe der Formel II

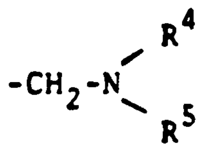

(II) ,

$R^2$ ein Wasserstoffatom oder einen Alkylrest mit 1 bis 3 C-Atomen und
$R^3$ ein Wasserstoffatom, eine geradkettige oder verzweigte Alkylgruppe mit 1 bis 4 C-Atomen, Hydroxymethyl oder eine Aminomethylgruppe der Formel II bedeuten, wobei
$R^4$ und $R^5$ gleich oder verschieden sind und ein Wasserstoffatom oder einen geradkettigen oder verzweigten Alkylrest mit 1 bis 4 C-Atomen darstellen, oder beide Reste zusammen mit dem Stickstoffatom, an das sie gebunden sind, einen 5- bis 7-gliedrigen gesättigten Ring mit 4 bis 6 C-Atomen oder mit 4 oder 5 C-Atomen und zusätzlich einem weiteren Heteroatom in Form von O, S oder $NR^6$ bilden und $R^6$ die Bedeutung von Wasserstoff oder $(C_1-C_4)$-Alkyl hat,
sowie die physiologisch verträglichen Säureadditionssalze der Verbindungen mit dem Strukturelement der Formel II in den Positionen von $R^1$ und/oder $R^3$.

Wenn $R^4$ und $R^5$ zusammen mit dem N-Atom, an das sie gebunden sind, einen gesättigten Ring mit 4 oder 5 C-Atomen und zusätzlich einem weiteren weiteren Heteroatom bilden, müssen die Heteroatome durch mindestens 1 C-Atom voneinander getrennt sein.

Bevorzugt sind dabei solche Verbindungen der Formel I, bei denen entweder $R^1$ einen tert.-Butylrest oder eine Aminomethylgruppe der Formel II bedeutet oder $R^2$ und $R^3$ unabhängig voneinander für Wasserstoff oder Methyl stehen. Weiterhin sind diejenigen Verbindungen der Formel I besonders hervorzuheben, in denen $R^1$ eine tert.-Butylgruppe darstellt und gleichzeitig $R^2$ und $R^3$ unabhängig voneinander für Wasserstoff oder Methyl stehen, wie etwa das 3-(3,5-Di-tert.butyl-4-hydroxyphenyl)-7H-thiazolo[3,2-b][1,2,4]-triazin-7-on.
Als Alkylreste für die Gruppen $R^1$ und $R^3$ bis $R^6$ kommen Methyl, Äthyl, n-Propyl, Isopropyl, n-Butyl, Isobutyl, sek.Butyl und tert.Butyl und für die Gruppe $R^2$ Methyl, Äthyl, n-Propyl und Isopropyl in Frage. Geeignete cyclische Aminomethylgruppen für das Strukturelement der Formel II stellen Pyrrolidino-, Piperidino-, Hexamethylenimino-, Morpholino-, Thiomorpholino- sowie gegebenenfalls am zweiten Stickstoffatom alkyliertes Piperazino- und Homopiperazino-methyl dar.

Gegenstand der Erfindung ist ferner ein Verfahren zur Herstellung der neuen 7H-Thiazolo[3,2-b][1,2,4]-triazin-7-one.

Eine Ausführungsform a) besteht beispielsweise darin, daß man 3-Mercapto-2H-1,2,4-triazin-5-one der Formel III,

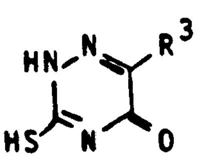

(III)

in der $R^3$ die oben genannte Bedeutung hat, mit 2-Halogen-1-phenylalkanonen der Formel IV,

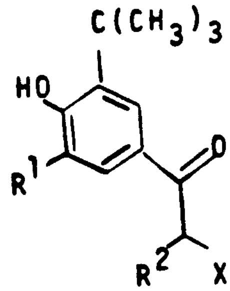

(IV)

in der $R^1$ die voranstehend definierte Alkylgruppe bedeutet, $R^2$ die oben angegebene Bedeutung hat und X für ein Halogenatom, vorzugsweise Chlor oder Brom steht, zu den entsprechenden erfindungsgemäßen Verbindungen der Formel I umsetzt.

Eine andere Ausführungsform b) besteht darin, daß man Verbindungen der Formel III mit einer Verbindung der Formel IV (mit $R^1$ = ($C_1$-$C_4$)-Alkyl) unter basischen Bedingungen zunächst zu den S-alkylierten 2H-1,2,4-Triazin-5-onen der Formel V

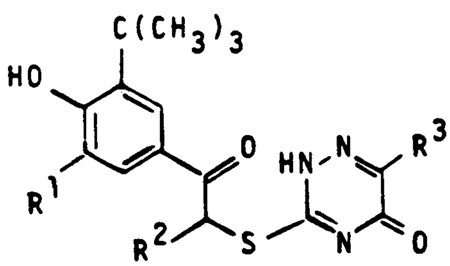

(V)

umsetzt und diese unter Dehydratisierung in die entsprechenden erfindungsgemäßen Verbindungen der Formel I umwandelt; die Zwischenprodukte der Formel V sind neue Verbindungen.

Eine weitere Ausführungsform c) besteht darin, daß man die 2-Halogen-1-phenylalkanone der Formel IV (Mit $R^1$ = ($C_1$-$C_4$)-Alkyl) zuerst mit Thiosemicarbazid zu den entsprechenden - ebenfalls neuen - 2-Amino-6H-1,3,4-thiadiazinen der Formel VI

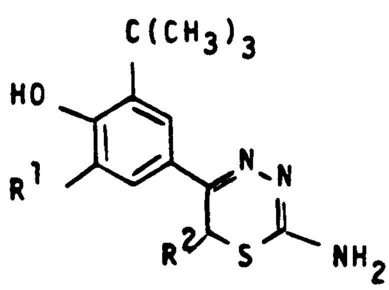

(VI)

umsetzt und diese unter sauren Bedingungen in die 3-Amino-2-imino-2,3-dihydro-thiazole der Formel VII

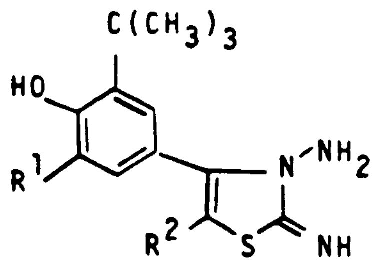

(VII)

umlagert, die anschließend mit α-Ketocarbonsäuren bzw. deren Alkylestern der Formel VIII,

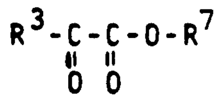

(VIII)

in der $R^3$ ein Wasserstoffatom, eine geradkettige oder verzweigte Alkylgruppe mit 1 bis 4 C-Atomen oder eine Hydroxymethylgruppe und $R^7$ ein Wasserstoffatom oder einen Alkylrest mit 1 bis 3 C-Atomen darstellen, zu den betreffenden erfindungsgemäßen Verbindungen der Formel I cyclokondensiert werden.

Eine ebenfalls praktikable Ausführungsform d) besteht darin, daß man ausgehend von jenen erfindungsgemäßen Verbindungen der Formel I, die in der Position von $R^1$ und/oder $R^3$ einen Hydroxymethyl-Rest tragen, zunächst deren Hydroxygruppe(n) entweder gegen Halogen austauscht oder in einen aktivierten Sulfon-oder Phosphorsäureester überführt und anschließend mit den Aminen der Formel IX,

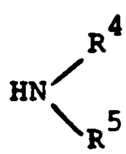

(IX)

in der $R^4$ und $R^5$ die zuvor genannten Bedeutungen haben, zu erfindungsgemäßen Verbindungen der Formel I mit dem Strukturelement der Formel II in der Stellung von $R^1$ und/oder $R^3$ umsetzt, wobei diese Aminomethyl-Verbindungen entweder in freier Form isoliert oder aber mit geeigneten Säuren in physiologisch verträgliche Additionssalze umgewandelt werden.

Eine weitere für die Herstellung von Verbindungen der Formel I mit dem Strukturmerkmal der Formel II oder dem Hydroxymethyl-Rest in der Position von $R^1$ geeignete Ausführungsform e) besteht schließlich darin, daß man Verbindungen der Formel I, in der $R^1$ für Wasserstoff steht,
zur Einführung des Hydroxymethyl-Restes mit Formaldehyd umsetzt,
zur Einführung von N-substituierten Aminomethylgruppen der Formel II, in der also $R^4$ und $R^5$ nicht gleichzeitig Wasserstoff bedeuten, mit den entsprechenden Aminen der Formel IX in Gegenwart von Formaldehyd umsetzt oder
zur Einführung des unsubstituierten Aminomethyl-Restes ($R^4$ = $R^5$ = H) zunächst mit N-Hydroxymethyl-acetamiden der Formel X,

$R^8$-CO-NH-$CH_2$-OH (X)

in der $R^8$ für Trifluormethyl, Trichlormethyl oder Chlormethyl steht, unter saueren Bedingungen und Wasseraustritt kondensiert und anschließend den Acylrest $R^8$-CO- hydrolytisch eliminiert, wobei die so erhaltenen Aminomethyl-Verbindungen entweder in freier Form isoliert oder aber mit geeigneten Säuren in physiologisch verträgliche Additionssalze überführt werden.

Für die Herstellung dieser Säureadditionsssalze kommen beispielsweise Mineralsäuren, wie Schwefel- oder Phosphorsäure oder Halogenwasserstoffsäuren, insbesondere Bromwasserstoff-und Salzsäure, sowie organische Säuren, wie ein- bis dreibasische Carbonsäuren, z.B. Essig-, Milch-, Malein-, Fumar-, Oxal-,

Wein-, Zitronensäure oder andere verträgliche Säuren, wie Sulfonsäuren (Benzolsulfonsäure, 4-Toluolsulfonsäure, Methansulfonsäure, Trifluormethylsulfonsäure, Cyclohexylamidosulfonsäure, etc.) in Frage.

Die für das erfindungsgemäße Verfahren als Ausgangsstoffe benutzten 3-Mercapto-2H-1,2,4-triazin-5-one der Formel III sind bekannt (H. Neunhoeffer und P. L. Wiley in A. Weisberger und E. C. Taylor, The Chemistry of Heterocyclic Compounds, Wiley-Interscience, New York, Bd. 33 (1978) S. 430 - 465) oder lassen sich auf analoge Weise darstellen.

Die ebenfalls als Ausgangsstoffe verwendeten 2-Halogen-1-phenylalkanone der Formel IV sind auch literaturbekannt oder lassen sich beispielsweise aus den 1-(3-Alkyl-5-tert.-butyl-4-hydroxyphenyl)-alkanonen durch Umsetzung mit einem geeigneten Halogenierungsmittel nach den im Houben-Weyl Bd. V/4 S. 171 - 189 (1960) beschriebenen Methoden leicht herstellen.
Als geeignete Verbindungen IV seien beispielsweise das 2-Brom-1-(3,5-di-tert.butyl-4-hydroxyphenyl)-äthanon und 2-Brom-1-(3-methyl-5-tert.butyl-4-hydroxyphenyl)-äthanon genannt, die sich durch Halogenierung der entsprechend substituierten 1-Phenylalkanone mit elementarem Brom oder mit Kupfer-II-bromid nach einem Verfahren von L. C. King und G. K. Ostrum, J. org. Chem. 29 (1964) S. 3459 - 3461 herstellen lassen.

Zur Gewinnung jener Verbindungen der Formel IV, in denen für X ein Chloratom steht, eignet sich insbesondere Sulfurylchlorid, das vorzugsweise bei Temperaturen zwischen etwa 10 und 30 °C in Anwesenheit inerter Lösungsmittel wie z.B. Methylenchlorid oder Chloroform, mit den entsprechenden 1-Phenylalkanonen zur Reaktion gebracht wird. Ein weiteres Herstellungsverfahren besteht in der Friedel-Crafts-Acylierung von 2-Alkyl-6-tert.butylphenolen mit vorzugsweise Chloracetylchlorid in Gegenwart von Lewissäuren, wie z. B. Aluminiumchlorid oder Bortrifluorid.

Bei der Umsetzung der 3-Mercapto-2H-1,2,4-triazin-5-one III mit den 2-Halogen-1-phenylalkanonen IV gemäß Verfahrensweise a) arbeitet man gewöhnlich mit äquimolaren Mengen der Reaktionspartner in einem Verteilungs-oder Lösungsmittel. Als solche kommen vor allem polare Solventien, beispielsweise niedere aliphatische Carbonsäuren, wie Ameisensäure oder Essigsäure, Alkohole,wie Methanol, Äthanol, die verschiedenen Propanole oder Butanole, in Frage. Es können aber auch Äthylenglykol und dessen Äther, Essigsäureester, Aceton, Butan-2-on, Dimethylformamid oder Acetonitril sowie Mischungen der genannten Lösungsmittel oder deren Gemische mit Wasser Verwendung finden. Die Reaktionstemperaturen liegen im allgemeinen zwischen etwa 20 °C und dem Siedepunkt des jeweils verwendeten Reaktionsmediums. Vorzugsweise wird in Essigsäure zwischen etwa 70 und 100 °C gearbeitet, wobei die Reaktionszeiten im allgemeinen zwischen weniger als einer Stunde und etwa 6 Stunden liegen.

Die Herstellung der bei Verfahrensvariante b) als Zwischenprodukte benötigten S-alkylierten 2H-1,2,4-Triazin-5-one der Formel V kann in der Weise erfolgen, daß man 3-Mercapto-2H-1,2,4-triazin-5-one der Formel III zweckmäßigerweise mit einer äquimolaren Menge eines 2-Halogen-1-phenylalkanons der Formel IV in Gegenwart eines basischen Mittels, wie eines Alkali- oder Erdalkalihydroxids, -carbonats, -hydrids oder -alkoholats,oder einer organischen Base, beispielsweise eines Trialkylamins, wie Triäthyl- oder Tributylamin, umsetzt. Die Reaktion erfolgt bevorzugt in einem gegenüber den Reaktionspartnern indifferenten Verteilungs- oder Lösungsmittel oder deren Gemischen.Als solche kommen beispielsweise Wasser, Alkohole,wie Methanol, Äthanol, die verschiedenen Propanole und Butanole, Äther,wie Diäthyläther, Diisopropyläther, Tetrahydrofuran und Dioxan, Nitrile, wie Acetonitril, Ketone, wie Aceton und Butanon,sowie Dimethylformamid, Dimethylacetamid und Dimethylsulfoxid in Frage. Die Umsetzung wird in der Regel zwischen etwa 0 ° und der Siedetemperatur des Reaktionsmediums, vorzugsweise zwischen etwa 30 und 90 °C durchgeführt, wobei Reaktionszeiten von durchschnittlich einer bis zu mehreren Stunden benötigt werden.

Die anschließende Dehydratisierung der Verbindungen V unter Ringschluß zu den erfindungsgemäßen Verbindungen der Formel I gelingt bevorzugt in Gegenwart einer Säure, wie Chlorwasserstoff- oder Bromwasserstoff-, Schwefel-, p-Toluolsulfon-, Polyphosphor- oder Essigsäure in einem Äther, wie Diisopropyläther, Tetrahydrofuran und Dioxan,oder Alkohol, wie Methanol, Äthanol oder Propanol, oder deren Gemischen mit Wasser bei Temperaturen von etwa 0 bis 80 °C, insbesondere von etwa 10 bis 40 °C, und Reaktionszeiten von etwa einer Stunde bis zu mehreren Tagen.

Die Verfahrensweise c) führt über die 2-Amino-6H-1,3,4-thiadiazine der Formel VI als Zwischenprodukte, die sich aus den 2-Halogen-1-phenylalkanonen der Formel IV und Thiosemicarbazid nach Literaturmethoden leicht herstellen lassen. Deren Umlagerung in die entsprechenden 3-Amino-2-imino-2,3-dihydrothiazole der Formel VII erfolgt vorzugsweise in einem sauren Medium, wie beispielsweise in Chlorwasserstoff-, Bromwasserstoff-, Schwefel- oder Essigsäure, sowie deren Mischungen oder Gemischen mit Wasser.

Die anschließende Cyclokondensation zu den Verbindungen der Formel I wird im allgemeinen ohne Zwischenisolierung der intermediär gebildeten 3-Amino-2-imino-2,3-dihydro-thia= zole VII durchgeführt, indem man der Reaktionsmischung eine α-Ketocarbonsäure oder deren Alkylester der Formel VIII, wie etwa

Glyoxylsäure oder Brenztraubensäure bzw. deren Methyl- oder Äthylester, in bis zu zweifach äquimolarer Menge hinzufügt. Die Temperaturen liegen dabei vorzugsweise zwischen etwa 50 ° C und dem Siedepunkt des jeweils verwendeten Reaktionsmediums, während die Reaktionszeiten im allgemeinen von etwa 5 bis zu etwa 30 Stunden betragen können.

Die etwaige Umwandlung der erfindungsgemäßen Verbindungen der Formel I, die in der Position von $R^1$ und/oder $R^3$ einen Hydroxymethyl-Rest tragen, in die Aminomethyl-Verbindung der Formel I entsprechend Ausführungsform d) erfolgt in üblicher Weise. So kann die Atkiviertung der Hydroxylgruppe beispielsweise durch Umsetzung mit Halogenierungsmitteln, wie Thionylchlorid und Phosphortrichlorid oder -bromid, zu den Halogen= methyl—Verbindungen oder durch Veresterung z. B. mit Methan-oder Toluol-4-sulfonylchlorid vorgenommen werden. Bei der anschließenden Kondensationsreaktion mit den Aminen der Formel IX arbeitet man vorteilhaft in Gegenwart einer mindestens zweifach molaren Menge des jeweils eingesetzten Amins pro derivatisierter Hydroxymethylgruppe ; auch der Einsatz äquivalenter Mengen beider Reaktionspartner ist möglich, doch empfiehlt sich dann die Zugabe eines säurebindenden Mittels, z.B. eines Alkali- oder Erdalkalihydroxids oder -carbonats oder auch einer organischen Base, wie Tri= äthylamin oder Pyridin,in mindestens stöchiometrischer Menge. Die Umsetzung wird zweckmäßig in einem gegenüber den Reaktionspartnern inerten Lösungs- oder Verteilungsmittel durchgeführt. Hierfür kommen z. B. Alkohole, wie Methanol, Äthanol, Iso= propanol, n-Propanol, die verschiedenen Butanole, sowie Gemische derselben, oder auch deren Mischungen mit Äthern, wie Tetra= hydrofuran und Dioxan, oder Kohlenwasserstoffen, wie Benzol, Toluol und Xylol, sowie aprotische Lösungsmittel, wie Pyridin, Dimethylformamid, Dimethylacetamid, Dimethylsulfoxyd und Hexamethylphosphorsäuretriamid in Frage. Die Reaktion wird im allgemeinen bei Temperaturen zwischen etwa 0 ° C und dem Siedepunkt des jeweiligen Lösungsmittels, vorzugsweise zwischen etwa 20 und 100 ° C durchgeführt, wobei die Reaktionszeit bis zu mehreren Stunden betragen kann.

Zur Einführung der Aminomethylgruppen gemäß Formel II in die Verbindungen der Formel I, in der $R^1$ für Wasserstoff steht, entsprechend Verfahrensweise e) bedient man sich im Falle der Umsetzungen mit primären und insbesondere sekundären Aminen der Formel IX vorteilhaft der aus der Literatur hinlänglich bekannten Mannich-Reaktion (z. B. Houben-Weyl, Bd. XI/1 (1957), S. 755 - 763). Der an der Umsetzung beteiligte Formaldehyd kann sowohl in monomerer Form als wäßrige Lösung oder in polymerer Form als Feststoff (z. B. Paraformaldehyd) eingesetzt werden. Im allgemeinen arbeitet man mit einer 4- bis 10-fach molaren Menge an Formaldehyd und einem bis zu 40-fach molaren Überschuß an dem jeweiligen Amin, das auch in Form seiner Hydrohalogenide zur Reaktion gebracht werden kann. Als Reaktionsmedium dienen bevorzugt Wasser oder Alkohole, wie Methanol, Ethanol oder Propanol, oder deren Gemische. Die Umsetzung erfolgt vorzusgweise bei Temperaturen zwischen etwa 20 ° und 100 ° C, wobei Reaktionszeiten von einer Stunde bis zu mehreren Tagen benötigt werden.

Die Kondensation mit den N-Hydroxymethylacetamiden der Formel X bietet den bevorzugten Weg zur Einführung des am Stickstoffatom unsubstituierten Aminomethyl-Restes. Als Kondensationsmittel kommen Säuren, wie etwa Methansulfonsäure oder Gemische von konzentrierter Schwefelsäure und Eisessig, in Frage, die gleichzeitig als Reaktionsmedium dienen können, wobei die Umsetzung in der Regel zwischen etwa 0 ° C und Raumtemperatur abläuft und innerhalb von 30 Minuten bis 6 Stunden beendet ist. Die Abspaltung des Acylrestes aus den primär gebildeten N-acylierten Aminomethylverbindungen erfolgt nach dem Fachmann hinlänglich bekannten Standardverfahren durch saure Hydrolyse, wobei sich das Arbeiten in wäßriger Chlorwasserstoff-, Bromwasserstoff-oder Schwefelsäure bei erhöhter Temperatur, vorzugsweise beim Siedepunkt des jeweiligen Reaktionsmediums, besonders bewährt.

Die erfindungsgemäßen 7H-Thiazolo[3,2-b][1,2,4]-triazin-7-one der Formel I und deren entsprechende Säureadditionssalze eignen sich aufgrund ihrer wertvollen pharmakologischen Eigenschaften bei gleichzeitig hervorragender Verträglichkeit in besonderer Weise für die Verwendung als Wirkstoffe in Arzneimitteln, insbesondere in solchen zur Behandlung von entzündlich rheumatischen Erkrankungen. Sie können etweder für sich allein, beispielsweise in Form von Mirkokapseln, in Mischungen miteinander oder in Kombination mit geeigneten Hilfs- und/oder Trägerstoffen verabreicht werden.

Gegenstand der Erfindung sind somit auch Arzneimittel, die aus mindestens einer Verbindung der Formel I und/oder mindestens einem von deren entsprechenden Säureadditionssalzen bestehen oder mindestens einen dieser Wirkstoffe neben pharmazeutisch geeigneten und physiologisch verträglichen Trägerstoffen, Verdünnungsmitteln und/oder anderen Hilfsstoffen enthalten.

Die erfindungsgemäßen Arzneimittel können oral, topisch, rektal oder gegebenenfalls auch parenteral appliziert werden, wobei die orale Anwendung bevorzugt ist.

Geeignete feste oder flüssige galenische Zubereitungsformen sind beispielsweise Granulate, Pulver, Dragees, Tabletten, (Mirko)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung üblicherweise Hilfsmittel, wie Trägerstoffe, Spreng-, Binde-,Überzugs-, Quellungs-, Gleit- oder Schmiermittel, Geschmacksstoffe, Süßungsmittel oder Lösungsvermittler, Verwendung finden. Als häufig verwendete Hilfsstoffe seien z. B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyäthylenglykole und Lösungsmittel, wie etwa steriles Wasser und ein- oder mehrwertige Alkohole, z.B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis mindestens einer Verbindung gemäß Formel I und/oder mindestens eines entsprechenden Säureadditionssalzes enthält. Bei festen Dosierungseinheiten, wie Tabletten, Kapseln, Dragees oder Suppositorien, kann diese Dosis bis zu etwa 800 mg, bevorzugt jedoch etwa 100 bis 500 mg betragen.

Für die Behandlung eines an entzüdlich rheumatischen Erkrankungen leidenden, erwachsenen Patienten sind - je nach Wirksamkeit der Verbindungen gemäß Formel I und/oder der entsprechenden Säureadditionssalze am Menschen - Tagesdosen von etwa 100 bis 2000 mg Wirkstoff, vorzugsweise etwa 300 bis 1000 mg, bei oraler Verabreichung indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Schließlich können die Verbindungen der Formel I und die entsprechenden Säureadditionssalze bei der Herstellung der vorgenannten galenischen Zubereitungsformen auch zusammen mit anderen geeigneten Wirkstoffen, beispielsweise Antiuricopathika, Thrombocytenaggregationshemmern, Analgetika und anderen steroidalen oder nicht-steroidalen Antiphlogistika, formuliert werden.

Die Struktur aller nachstehend beschriebenen Verbindungen wurde durch Elementaranalyse und IR- sowie $^1$H-NMR-Spektren gesichert. Die gemäß den nachfolgenden Beispielen 1 bis 5 bzw. 12 und 13 sowie die auf analoge Weise hergestellten Verbindungen der Formel I sind in Tabelle 1 zusammengefaßt.

Beispiel 1: 3-(3,5-Di-tert.butyl-4-hydroxyphenyl)-7H-thiazolo[3,2-b][1,2,4]triazin-7-on

nach Verfahrensweise a)

$a_1$) 2-Brom-1-(3,5-di-tert.butyl-4-hydroxyphenyl)-äthanon

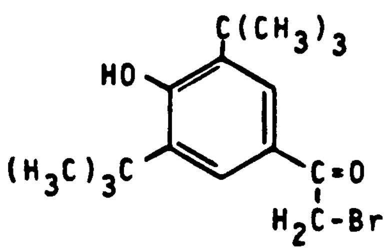

206 g (0,83 mol) 1-(3,5-Di-tert.butyl-4-hyroxyphenyl)-äthanon wurden unter Rühren in 415 ml Methylenchlorid gelöst, zum Sieden erhitzt und innerhalb von 30 Minuten tropfenweise mit 144 g (0.9 mol) Brom versetzt. Danach wurde für weitere 2 Stunden unter Rückfluß erhitzt, die Mischung abgekühlt, mit 400 ml Wasser versetzt, die organische Phase abgetrennt und über Natriumsulfat getrocknet. Nach dem Entfernen des Lösungsmittels unter vermindertem Druck wurde das erhaltene feste Rohprodukt aus 540 ml Methylcyclohexan umkristallisiert.

Ausbeute:

191 g (67 % der Theorie)
Schmelzpunkt: 105 - 108° C
$C_{16}H_{23}BrO_2$ (MG = 327,3)

$a_2$) 3-(3,5-Di.tert.butyl-4-hydroxyphenyl)-7H-thiazolo [ 3,2-b][1,2,4]triazin-7-on

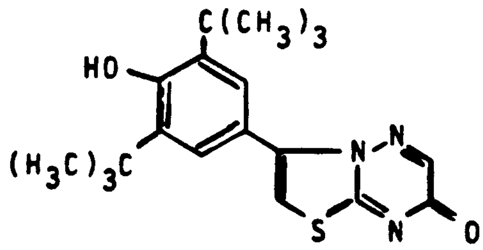

197 g (0,6 mol) 2-Brom-1-(3,5-di-tert.butyl-4-hydroxyphenyl)-äthanonaus Stufe $a_1$) und 80 g (0,62 mol) 3-Mercapto-2H-1,2,4-triazin-5-on wurden in 700 ml Eisessig 4 Stunden bei 90° C gerührt. Danach ließ man die Reaktionsmischung langsam erkalten und saugte den gebildeten kristallinen Niederschlag ab, der anschließend mit Wasser gewaschen und dann in 1000 ml Wasser bei maximal 90° C für 30 Minuten ausgerührt wurde. Die Umkristallisation des noch wasserfeuchten Kristallbreies erfolgte aus 7000 ml Äthanol.

Ausbeute:

171,6 g (80 % der Theorie)
Schmelzpunkt: 257° C(Zers.)
$C_{19}H_{23}N_3O_2S$ (MG = 357,5)

| Analyse: | | | | |
|---|---|---|---|---|
| Berechnet: | C 63,84 % | H 6,49 % | N 11,75 % | S 8,99 % |
| Gefunden: | C 63,55 % | H 6,44 % | N 12,03 % | S 9,00 % |

nach Verfahrensweise b)

$b_1$) 3-[(3,5-Di-tert.butyl-4-hydroxyphenacyl)-thio]-2H-1,2,4-triazin-5-on

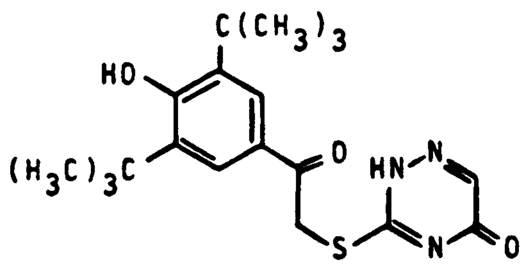

Zu einer Suspension von 12,9 g (0,1 mol) 3-Mercapto-2H-1,2,4-triazin-5-onin 250 ml Wasser gab man 5,3 g (0,05 mol) Natriumcarbonat und rührte 30 Minuten, tropfte dann eine Lösung von 32,7 g (0,1 mol) 2-Brom-1-(3,5-di-tert.butyl-4-hydroxyphenyl)-äthanon in 250 ml Methanol zu und hielt das Reaktionsgemisch für 1 Stunde auf 75° C. Nach dem Abkühlen filtrierte man den angefallenen Niederschlag ab und kristallisierte aus Essigsäureäthylester um.

Ausbeute:

26,6 g (71 % der Theorie)
Schmelzpunkt: 209 - 211° C
$C_{19}H_{25}N_3O_3S$ (MG = 375,5)

| Analyse: | | | | |
|---|---|---|---|---|
| Berechnet:<br>Gefunden: | C 60,78 %<br>C 60,45 % | H 6,71 %<br>H 6,82 % | N 11,19 %<br>N 11,06 % | S 8,54 %<br>S 8,68 % |

b₂) 3-(3,5-Di-tert.butyl-4-hydroxyphenyl)-7H-thiazolo[3,2-b] [1,2,4 ]triazin-7-on

3,7 g (0.01 mol) 3-[(3,5-Di-tert.butyl-4-hydroxyphenacyl)-thio]-2H-1,2,4-triazin-5-on aus Stufe $b_1$) wurden in einer Mischung aus 60 ml Tetrahydrofuran und 50 ml 2 N Salzsäure 36 Stunden bei Raumtemperatur gerührt. Das langsam ausgeschiedene Cyclokondensationsprodukt wurde abfiltriert und ein- bis dreimal aus Tetrahydrofuran/Äthanol (3 : 2) umkristallisiert.

Ausbeute:

1,5 g (42 % der Theorie)
Schmelzpunkt: 256 - 257° (Zers.)
$C_{19}H_{23}N_3O_2S$ (MG = 357,5)
Analytische und spektroskopische Daten bestätigten die Identität des gewonnenen Produktes mit der nach Verfahrensweise a) hergestellten Verbindungen.

nach Verfahrensweise c)

$c_1$) 2-Amino-5-(3,5-di-tert.butyl-4-hydroxyphenyl)-6H-1,2,4-thiadiazin-hydrobromid

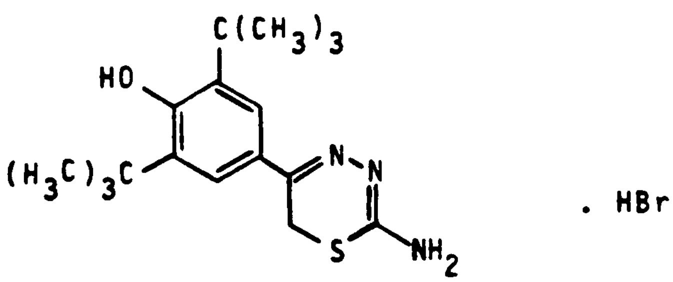

Eine Lösung von 32,7 g (0,1 mol) 2-Brom-1-(3,5-di-tert.butyl-4-hydroxyphenyl)-äthanon (Beispiel $1a_1$) und 9,1 g (0,1 mol) Thiosemicarbazid in 250 ml Eisessig wurde 1 Stunde bei Raumtemperatur gerührt und der ausgeschiedene Niederschlag abfiltriert und in 500 ml Äthanol heiß gelöst. Nach dem Abkühlen wurden die Kristalle abgesaugt, mehrmals mit Essigsäureäthylester gewaschen und im Vakuum getrocknet.

Ausbeute:

27,2 g (68 % der Theorie)
Schmelzpunkt: 255 - 257° C
$C_{17}H_{26}BrN_3OS$ (MG = 400,4)

| Analyse: | | | | | |
|---|---|---|---|---|---|
| Berechnet:<br>Gefunden: | C 51,00 %<br>C 50,71 % | H 6,55 %<br>H 6,52 % | Br 19,96 %<br>Br 19,92 % | N 10,49 %<br>N 10,46 % | S 8,01 %<br>S 8,17 % |

$c_2$) 3-(3,5-Di-tert.butyl-4-hydroxyphenyl)-7H-thiazolo[3,2-b] [1,2,4]triazin-7-on

Zu einer Lösung von 10,0 g (0,025 mol) 2-Amino-5-(3,5-di-tert.butyl-4-hydroxyphenyl)-6H-1,3,4-thiadiazin-hydrobromid aus Stufe $c_1$) in 250 ml Eisessig und 25 ml 4 N Salzsäure wurden bei 80° C 4,8 g (0,05 mol) Glyoxylsäure-monohydrat in 75 ml Wasser zugetropft. Nach 20-stündigem Rühren bei 80° C wurde unter vermindertem Druck eingedampft. Der feste Rückstand ließ sich vorteilhaft durch Säulenchromatographie an Kieselgel mit Methylenchlorid/Methanol (25 : 1) als Fließmittel und anschließende Umkristallisation aus Äthanol reinigen und erwies sich aufgrund der analytischen und spektroskopischen Untersuchungen indentisch mit dem nach den Verfahrensweisen a) und b) hergestellten Produkt.

Ausbeute:

3,9 g (42 % der Theorie)
Schmelzpunkt: 255 - 256° C (Zers.)
$C_{19}H_{23}N_3O_2S$ (MG = 357,5)

Beispiel 2: 3-(3-tert.Butyl-5-methyl-4-hydroxyphenyl)-7H-thiazolo[3,2-b][1,2,4]-triazin-7-on

$a_1$) 2-Brom-1-(3-tert.butyl-5-methyl-4-hydroxyphenyl)-äthanon

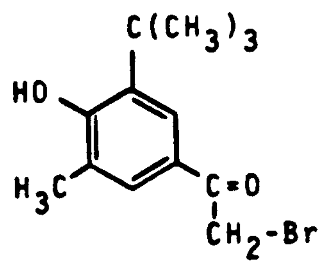

Eine zum Sieden erhitzte Suspension von 179 g (0,8 mol) Kupfer(II)bromid in 360 ml Essigsäureäthylester wurde unter Rühren tropfenweise mit einer Lösung von 82,5 g (0,4 mol) 1-(3-tert.Butyl-5-methyl-4-hydroxyphenyl)-äthanon in 360 ml Chloroform versetzt. Anschließend wurde bis zur Beendigung der Bromwasserstoff-Entwicklung 4 Stunden lang unter Rückfluß erhitzt. Nach dem Abkühlen auf Raumtemperatur wurden die Kupfersalze abgesaugt, der Filterrückstand mehrmals mit Essigsäureäthylester gewaschen, das Filtrat unter vermindertem Druck eingeengt und der feste Rückstand aus Cyclohexan umkristallisiert.

Ausbeute:

81,9 g (72 % der Theorie)
Schmelzpunkt: 90 - 92 ° C
$C_{13}H_{17}BrO_2$ (MG = 285,2)

$a_2$) 3-(3-tert.Butyl-5-methyl-4-hydroxyphenyl)-7H-thiazolo [3,2-b][1,2,4]triazin-7-on

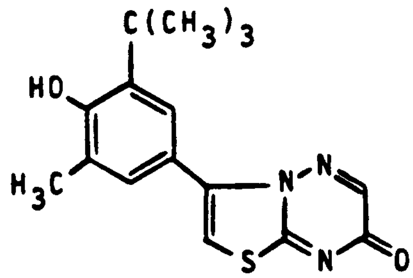

Eine Lösung aus 28,5 g (0,1 mol) 2-Brom-1-(3-tert.butyl-5-methyl-4-hydroxyphenyl)-äthanon aus Stufe $a_1$) und 12,9 g (0,1 mol) 3-Mercapto-2H-1,2,4-triazin-5-on wurden in 250 ml Äthanol 8 Stunden unter Rückfluß erhitzt. Nach dem Entfernen des Lösungsmittels im Vakuum wurde der feste Rückstand in 200 ml siedenden Essigsäureäthylesters gelöst und heiß filtriert. Beim Einengen des Filtrats fielen farblose Kristalle an, die nochmals aus Isopropanol umkristallisiert wurden.

Ausbeute:

18,9 g (61 % der Theorie)
Schmelzpunkt: 225 - 226° C (Zers.)
$C_{16}H_{17}N_3O_2S$ (MG = 315,4)

| Analyse: | | | | |
|---|---|---|---|---|
| Berechnet:<br>Gefunden: | C 59,23 %<br>C 59,51 % | H 8,08 %<br>H 8,17 % | N 12,95 %<br>N 12,83 % | S 9,88 %<br>S 9,82 % |

Beispiel 3: 3-(3,5-Di - tert.butyl-4-hydroxyphenyl)-6-hydroxymethyl-7H-thiazolo[3,2-b][1,2,4]triazin-7-on

$a_1$) 6-Hydroxymethyl-3-mercapto-2H-1,2,4-triazin-5-on

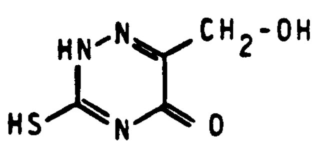

4,0 g (0,18 mol) Lithiumborhydrid wurden in 100 ml wasserfreien Tetrahydrofurans suspendiert und unter Rühren und Eiskühlung mit einer Lösung von 19,0 g (0,094 mol) 6-Äthoxycarbonyl-3-mercapto-2H-1,2,4-triazin-5-on in 40 ml absoluten Tetrahydrofurans tropfenweise versetzt. Danach wurde das Reaktionsgemisch 4 Stunden lang unter Rückfluß gekocht. Nach dem Abkühlen gab man unter weiterem Rühren portionsweise Wasser hinzu und stellte die Mischung nach Beendigung der Wasserstoff-Entwicklung mit 10 %iger Schwefelsäure auf pH 1 ein. Nach dem Abdampfen des Tetrahydrofurans unter vermindertem Druck wurde die Lösung mit Essigsäureäthylester mittels eines Perforators extrahiert. Eindampfen der über Natriumsulfat getrockneten organischen Phase und Umkristallisation des festen Rückstandes aus Wasser lieferte das Produkt in Form gelber Nadeln.

Ausbeute:

9,5 g (63 % der Theorie)
Schmelzpunkt: 233 - 235° C

$C_4H_5N_3O_2S$ (MG = 159,2)

$a_2$) 3-(3,5-Di-tert.butyl-4-hydroxyphenyl)-6-hydroxymethyl-7H-thiazolo[3,2-b][1,2,4]triazin-7-on

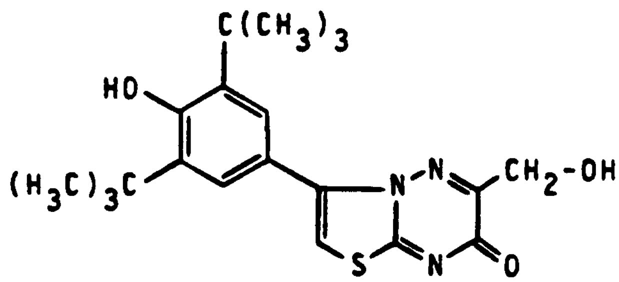

14,4 g (0,044 mol) 2-Brom-1-(3,5-di-tert.butyl-4-hydroxyphenyl)-äthanon (Beispiel $1a_1$) und 7,0 g (0,044 mol) 6-Hydroxymethyl-3-mercapto-2H-1,2,4-triazin-5-on aus Stufe $a_1$) werden in 300 ml Äthanol 4 Stunden zum Sieden erhitzt. Das unter reduziertem Druck eingeengte Reaktionsgemisch wurde in 300 ml Chloroform gelöst und mit 100 ml gesätiggter Natriumhydrogencarbonat-Lösung behandelt. Die Chloroformphase wurde schließlich abgetrennt, getrocknet und eingeengt. Filtration des Rohprodukts über Kieselgel mit Essigester/Methanol (99 : 1) als Fließmittel lieferte farblose Nadeln.

Ausbeute:

11,1 g (65 % der Theorie)
Schmelzpunkt: 215 - 217° C
$C_{20}H_{25}N_3O_3S$ (MG = 387,5)

| Analyse: | | | | |
|---|---|---|---|---|
| Berechnet: | C 61,99 % | H 6,50 % | N 10,84 % | S 8,27 % |
| Gefunden: | C 61,98 % | H 6,65 % | N 10,75 % | S 8,25 % |

Beispiel 4: 3-(3,5-Di-tert.butyl-4-hydroxyphenyl)-6-pyrrolidino= methyl-7H-thiazolo[3,2-b][1,2,4]triazin-7-on-hydrochlorid
(nach Verfahrensweise d))

$d_1$) 3-(3,5-Di-tert.butyl-4-hydroxyphenyl)-6-chlormethyl-7H-thiazolo [3,2-b][1,2,4]triazin-7-on

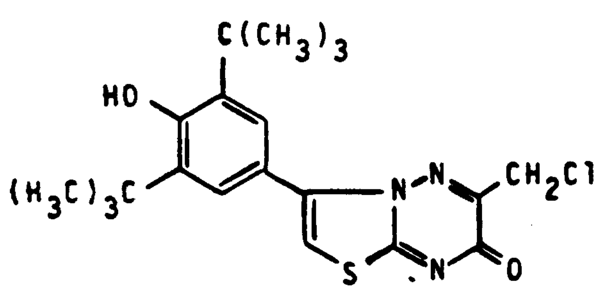

9,5 g (0,025 mol) des Thiazolo-traizinons gemäß vorgenanntem Beispiel $3a_2$ wurden in 230 ml trockenem Methylenchlorid gelöst und nach Zugabe von 2,1 ml Pyridin mit 7,0 g (0,06 mol) Thionylchlorid tropfenweise versetzt. Nach einstündigem Erhitzen unter Rückfluß und Abkühlen auf Raumtemperatur fügte man 200 ml Diäthyläther hinzu und ließ im Kühlschrank über Nacht stehen. Das ausgefallene Kristallisat

wurde abgesaugt und im Vakuum getrocknet.

Ausbeute:

6,7 g (66 % der Theorie)
Schmelzpunkt: 238 - 240° C
$C_{20}H_{24}ClN_3O_2S$ (MG = 405,9)

d₂) 3-(3,5-Di-tert.butyl-4-hydroxyphenyl)-6-pyrrolidino= methyl-7H-thiazolo[3,2-b][1,2,4]traizin-7-on-hydrochlorid

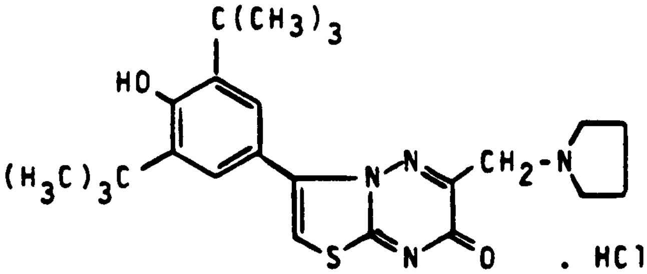

Eine Lösung von 6,7 g (0,017 mol) der Chlormethyl-Verbindung aus Stufe $d_1$)in 100 ml Methylenchlorid wurde nach Zugabe von 2,5 g (0,035 mol) Pyrrolidin 2,5 Stunden zum Sieden erhitzt, danach abgekühlt,zweimal mit Wasser gewaschen, über Natriumsulfat getrocknet und zur Bildung des Hydrochlorids eine äquimolare Menge äthanolischer Salzsäure hinzugegeben. Das kirstallin angefallene Rohprodukt wurde abfiltriert und aus einem Isopropanol/Essigsäureäthylester-Gemisch (1:1) umkristallisiert.

Ausbeute:

4,2 g (52 % der Theorie)
Schmelzpunkt: 222 - 223° C
$C_{24}H_{33}ClN_4O_2S$ (MG = 477,1)

| Analyse: | | | | | |
|---|---|---|---|---|---|
| Berechnet:<br>Gefunden: | C 60,42 %<br>C 60,46 % | H 6,97 %<br>H 7,25 % | Cl 7,43 %<br>Cl 7,24 % | N 11,74 %<br>N 11,49 % | S 6,72 %<br>S 6,45 % |

Beispiel 5 3-(3,5-Di-tert.butyl-4-hydroxyphenyl)-2,6-dimethyl-7H-thiazolo[3,2-b][1,2,4]triazin-7-on

$a_1$) 2-Brom-1-(3,5-di-tert.butyl-4-hydroxyphenyl)-propanon

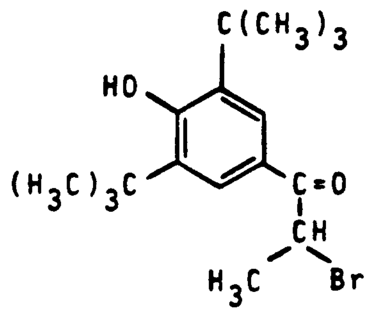

Zu einer siedenden Suspension von 139,0 g (0,62 mol) Kupfer-II-bromid in 300 ml Essigsäureäthylester tropfte man unter Rühren eine Lösung von 82,0 g (0,31 mol) 1-(3,5-Di-tert.butyl-4-hydroxyphenyl)-propanon in 300 ml Chloroform. Anschließend wurde bis zur Beendigung der Bromwasserstoff-Entwicklung 3 Stunden unter Rückfluß erhitzt. Nach Abkühlen auf Raumtemperatur wurden die Kupfersalze abgesaugt, der Filterrückstand zweimal mit Essigsäureäthylester nachgewaschen und das Filtrat unter vermindertem Druck eingeengt. Die Umkristallisation des festen Rückstandes erfolgte aus Petroläther (40 - 60° C).

Ausbeute:

87,5 g (82 % der Theorie)
Schmelzpunkt: 130 - 132° C
$C_{17}H_{25}BrO_2$ (MG = 341,3)

$a_2$) 3-(3,5-Di-tert.butyl-4-hydroxyphenyl)-2,6-dimethyl-7H-thiazolo[3,2-b][1,2,4]triazin-7-on

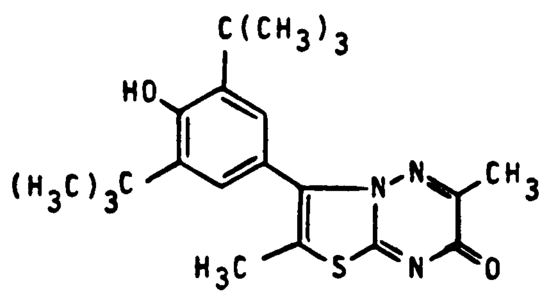

17,1 g (0,05 mol) 2-Brom-1-(3,5-di-tert.butyl-4-hydroxy= phenyl)-propanon aus Stufe $a_1$) und 7,2 g (0,05 mol) 3-Mercapto-6-methyl-2H-1,2,4-triazin-5-on wurden in 60 ml Eisessig 4 Stunden bei 90° C gerührt. Das unter reduziertem Druck eingedampfte Reaktionsgemisch wurde in 300 ml Chloroform gelöst und mit 100 ml gesättigter Natriumhydrogencarbonat-Lösung behandelt. Nach Abtrennen, Trocknen und Einengen der Chloroformphase erfolgte zweimaliges Umkristallisieren aus Isopropanol.

Ausbeute:

10,8 g (56 % der Theorie)
Schmelzpunkt: 256 - 257° C
$C_{21}H_{27}N_3O_2S$ (MG = 385,5)

| Analyse: | | | | |
|---|---|---|---|---|
| Berechnet:<br>Gefunden: | C 65,42 %<br>C 65,11 % | H 7,06 %<br>H 7,15 % | N 10,90 %<br>N 10,75 % | S 8,32 %<br>S 8,16 % |

Beispiel 12: 3-(3-Amionomethyl-5-tert.butyl-4-hydroxyphenyl)-7H-thiazolo[3,2-b][1,2,4]triazin-7-on-hydrochlorid
(nach Verfahrensweise e))

$e_1$) 3-(3-Tert.butyl-4-hydroxyphenyl)-7H-thiazolo[3,2-b] [1,2,4]triazin-7-on-hydrobromid

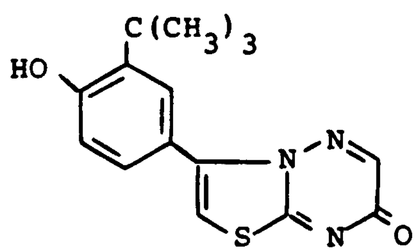

• HBr

Gemäß Verfahrensweise $a_2$) wurden 47.5 g (0.175 mol) 2-Brom-1-(3-tert.butyl-4-hydroxyphenyl)-äthanon und 20,6 g (0.16 mol) 3-Mercapto-2H-1,2,4-triazin-5-on in 190 ml Eisessig 1 Stunde bei 90° C gerührt. Der beim Erkalten anfallende Niederschlag wurde abgesaugt und aus einem Gemisch von Essigsäureäthylester/Äthanol umkristallisiert.

Ausbeute:

44.0 g (72 % der Theorie)
Schmelzpunkt: 250 - 252° C (Zers.)
$C_{15}H_{16}BrN_3O_2S$ (MG = 382.3)

| Analyse: | | | | | |
|---|---|---|---|---|---|
| Ber.:<br>Gef.: | C 47.13 %<br>C 47.22 % | H 4.22 %<br>H 4.25 % | Br 20.89 %<br>Br 20.43 % | N 10.98 %<br>N 11.17 % | S 8.36 %<br>S 8.60 % |

$e_2$) 3-(3-Aminomethyl-5-tert.butyl-4-hydroxyphenyl)-7H-thiazolo [3,2-b][1,2,4]traizin-7-on-hydrochlorid

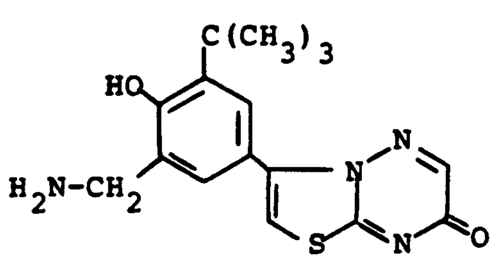

• HCl

Eine auf 5° C gekühlte Lösung von 15.1 g (0.05 mol) 3-(3-Tert.butyl-4-hydroxyphenyl)-7H-thiazolo[3,2-b][1,2,4] triazin-7-on in 240 ml Methansulfonsäure wurde portionsweise mit 7.1 g (0.005 mol) 2,2,2-Trifluor-N-(hydroxymethyl)-acetamid versetzt und anschließend 3 Stunden bei Raumtemperatur weitergerührt. Danach wurde die Reaktionsmischung in 1 l Eiswasser eingerührt und der Kristallbrei abgesaugt.

Zur Abspaltung der Trifluoracetylgruppe erhitzte man die erhaltene N-acylierte Aminomethyl-Verbindung in 600 ml 6 N-Salzsäure 1 Stunde unter Rückfluß. Die salzsaure Lösung wurde heiß filtriert, das Filtrat zur Trockne eingedampft und der Rückstand aus Essigsäurethylester/Methanol fraktioniert umkristallisiert.

Ausbeute:

8.4 g (46 % der Theorie)
Schmelzpunkt: 210 - 212° C
$C_{16}H_{19}ClN_4O_2S$ (MG = 366,9)

| Analyse: | | | | | |
|---|---|---|---|---|---|
| Ber.: | C 52.38 % | H 5.22 % | Cl 9.66 % | N 15,27 % | S 8.74 % |
| Gef.: | C 51.55 % | H 5.40 % | Cl 9.17 % | N 14.98 % | S 8.54 % |

Beispiel 13: 3-(3-Dimethylaminomethyl-5-tert.butyl-4-hydroxyphenyl)-7H-thiazolo[3,2-b][1,2,4] triazin-7-on-hydrochlorid
(nach Verfahrensweise e))

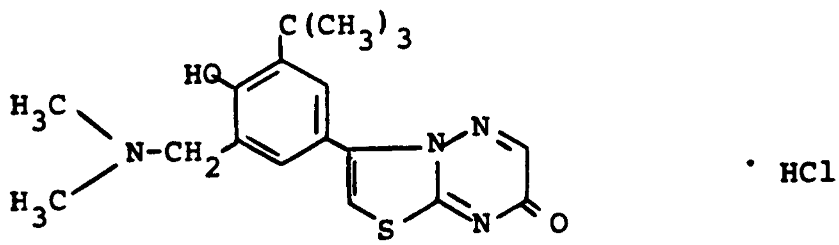

12.8 ml (0.28 mol) einer 40 %-igen wäßrigen Dimethylamin-Lösung wurden tropfenweise unter Eiskühlung zu 6.4 ml (0.086 mol) einer 37%igen wäßrigen Formaldehyd-Lösung gegeben. Nach Zusatz von 4.2 g (0.011 mol) 3-(3-Tert.butyl-4-hydroxyphenyl)-7H-thiazolo[3,2-b][1,2,4]triazin-7-on-hydrobromid aus Beispiel 12, Stufe $e_1$) und 70 ml Äthanol erhitzte man 19 Stunden zum Sieden. Nach dem Erkalten wurde 2 N Natronlauge zugetropft, bis sich die Mannich-Base abschied. Diese wurde abgesaugt, zur Bildung des Hydrochlorids eine äquimolare Menge äthanolischer Salzsäure hinzugegeben und abschließend aus Wasser umkristallisiert.

Ausbeute:

3.0 g (69 % der Theorie)
Schmelzpunkt: 260 - 261° C
$C_{18}H_{23}ClN_4O_2S$ (MG = 394.9)

| Analyse: | | | | | |
|---|---|---|---|---|---|
| Ber.: | C 54.75 % | H 5.87 % | Cl 8.98 % | N 14.19 % | S 8.12 % |
| Gef.: | C 54.58 % | H 5.89 % | Cl 9.22 % | N 13.90 % | S 8.11 % |

Tabelle 1: Verbindungen gemäß Formel I (siehe Anspruch 1)

| Beispiel | $R^1$ | $R^2$ | $R^3$ | Schmelzpunkt° C |
|---|---|---|---|---|
| 1 | $(H_3C)_3C$- | H | H | 257 (Zers.) |
| 2 | $H_3C$- | H | H | 225-226 (Zers.) |
| 3 | $(H_3C)_3C$- | H | $-CH_2-OH$ | 215-217 |
| 4 | $(H_3C)_3C$- | H | $-CH_2-$ 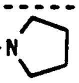 | 222-223 (als Hydrochlorid) |
| 5 | $(H_3C)_3C$- | $-CH_3$ | $-CH_3$ | 256-257 |
| 6 | $(H_3C)_3C$- | H | $-CH_3$ | 219-220 |
| 7 | $(H_3C)_3C$- | $-CH_3$ | H | 249-251 |
| 8 | $(H_3C)_3C$- | H | $-CH_2-CH_3$ | 240-242 |
| 9 | $(H_3C)_3C$- | H | $-CH_2-CH_2-CH_3$ | 216-217 |
| 10 | $(H_3C)_3C$- | H | $-CH(CH_3)_2$ | 237-238 |
| 11 | $H_3C$- | H | $-CH_3$ | 232-233 |
| 12 | $H_2N-CH_2$- | H | H | 210-212 (als Hydrochlorid) |
| 13 | $(H_3C)_3N-CH_2$- | H | H | 260-261 (als Hydrochlorid) |
| 14 | $(H_5C_2)_2N-CH_2$- | H | H | 234-236 (als Hydrochlorid) |
| 15 | 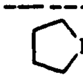 $-CH_2$- | H | H | 238-240 (als Hydrochlorid) |
| 16 | $-CH_2$- 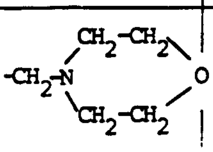 | H | H | 177-179 (als Hydrochlorid) |

Pharmakologische Prüfung und Ergebnisse

Die Prüfung der erfindungsgemäßen Verbindungen der Formel I auf antiphlogistische Wirkung, Beeinflussung immunpathologischer Prozesse, Sauerstoffradikale desaktivierende Potenz, ulzerogene Aktivität und akute Toxizität erfolgte in den anschließend beschriebenen Tiermodellen, wobei das in der Rheumatherapie zu den Standardpräparaten der ersten Wahl zählende Antiphlogistikum Naproxen (2-(6-Methoxy-2-naphthyl)-propionsäure) als Vergleichssubstanz in die Untersuchungen miteinbezogen wurde.

1. Adjuvans-Arthritis

Die Untersuchungen wurden nach der Methode von Pearson (Arthrit. Rheum. 2 (1959) S. 44) durchgeführt. Als Versuchstiere dienten männliche Ratten eines Wistar-Lewis-Stammes mit einem Körpergewicht zwischen 130 und 200 g. Die zu prüfenden Verbindungen wurden in Dosen von 50 mg pro kg Körpergewicht einmal täglich vom 1. bis zum 5. Versuchstag oral (p. o.) appliziert. Die Tiere einer Kontrollgruppe erhielten nur das Vehikel. Jede Präparat- und die Kontrollgruppe umfaßte 8 Tiere. Als Wirkungskriterium diente die prozentuale Herabsetzung der Pfotenvolumenzunahme gegenüber jener der unbehandelten Kontrollgruppe.

2. Akute gastrale Ulzerogenität

Die Untersuchung erfolgte an jeweils 10 männlichen Sprague-Dawley Ratten mit durch Hungerstreß sensibilisierter Magenschleimhaut. Das Körpergewicht der Tiere lag zwischen 200 und 300 g. 48 Stunden vor Applikation der Prüfpräparate bis zum Töten der Tiere wurde bei freiem Zugang zum Trinkwasser das Futter entzogen. Die Ratten wurden 24 Stunden nach oraler Substanzgabe getötet, die Mägen entnommen, unter fließendem Wasser gereinigt und auf Schleimhautläsionen inspiziert. Als Ulzera galten alle makroskopisch sichtbaren Läsionen.

Bestimmt wurde die Anzahl der Tiere mit Ulzera je Dosis und daraus die $UD_{50}$-Werte, also jene Dosen, mit denen bei 50 % der Tiere Läsionen hervorgerufen wurden, nach Litchfield und Wilcoxon (J. Pharmacol. exp. Ther. 96 (1949) S. 99) berechnet.

3. Akute Toxizität

Die Bestimmung der $LD_{50}$-Werte erfolgte standardgemäß über die innerhalb von 7 Tagen bei NMRI-(Naval Medical Research Institute)-Mäusen (6 Tiere pro dosi) nach einmaliger intraperitonealer (i. p.) Gabe auftretende Mortalität.

Die Ergebnisse dieser Untersuchungen, die die Überlegenheit der erfindungsgemäßen Verbindungen der Formel I gegenüber dem Standardpräparat Naproxen eindeutig belegen, sind in der nachstehenden Tabelle 2 zusammengefaßt.

Tabelle 2: Antiphlogistische Wirkung, Ulzerogenität und akute Toxizität

| Verbindung aus Beispiel | Adjuvans-Arthritis (% Hemmung bei 50 mg/kg p.o.) | Akute Ulzerogenität $UD_{50}$ (mg/kg) | Akute Toxizität $LD_{50}$ (mg/kg) |
|---|---|---|---|
| 1 | 67 | > 400 *) | > 1200 |
| 2 | 41 | > 400 *) | > 1200 |
| 3 | 41 | > 200 *) | > 1200 |
| 6 | 50 | > 200 *) | > 1200 |
| 9 | 45 | > 200 *) | > 1200 |
| Naproxen | 55 | 23 | 500 |

*) jeweils verabreichte Höchstdosis

Aus der Dosiswirkungskurve im Modell der Adjuvans-Arthritis ergab sich beispielsweise für die Verbindung aus Beispiel 1 ein $ED_{50}$-Wert von 10,9 mg/kg, der deutlich günstiger als der entsprechende Vergleichswert von 17,5 mg/kg für das Standardpräparat Naproxen liegt. Bezogen auf die akute Ulzerogenität errechnet sich daraus durch die Quotientenbildung von $UD_{50}$ zu $ED_{50}$ eine therapeutische Breite von > 36,7 für die Verbindung des Beispiels 1, die beim Vergleichspräparat Naproxen nur 1,3 beträgt, womit besonders eindrucksvoll unterstrichen wird, welch hoher Stellenwert der außerordentlich guten gastralen Verträglichkeit der erfindungsgemäßen Verbindungen zukommt. Eine ebenso eindeutige Überlegenheit gegenüber der Vergleichsverbindung ergibt sich, wenn man bei der Berechnung der therapeutischen Breite die ermittelten $LD_{50}$-Werte zugrundelegt und den Quotienten $LD_{50}/ED_{50}$ bildet, der für die Verbindung des Beispiels 1 über 110 und für Naproxen bei 28,6 liegt.

Auch in weiteren speziellen Versuchen erwiesen sich die erfindungsgemäßen Verbindungen dem Standardpräparat Naproxen eindeutig überlegen.

4. Hemmung immunpathologischer Prozesse

Es ist heute allgemein anerkannt, daß der progrediente Verlauf der entzündlich rheumatischen Erkrankungen hauptsächlich durch Entgleisungen im Immunsystem verursacht wird und daß eine kausale Therapie nur mit solchen Medikamenten gelingen kann, die diese immunpathologischen Prozesse zu durchbrechen vermögen.

a) Adjuvans Arthritis

In dem unter Punkt 1 beschriebenen Rattenmodell der mit Freundschem Adjuvans induzierten Arthritis ist gewöhnlich die Immunaktivität der Lymphocyten gegenüber bestimmten Mitogenen, wie Concavalin-A, Phytohämagglutinin-A und Dextransulfat, drastisch herabgesetzt. Es wurde daher die stimulierende Wirkung auf diese stark supprimierte Immunantwort untersucht. Hierbei bewirkte beispielsweise die Verbindung aus Beispiel 1 nach oraler Verabreichung von 3,15 und 6,3 mg/kg eine weitgehende Normalisierung der Immunreaktivität, während das in Dosen bis zu 25 mg/kg geprüfte Naproxen wirkungslos war.

b) Typ II-Collagen-induzierte Arthritis

In diesem Versuche wurde die Arthritis an männlichen Wistar Ratten mit Collagen vom Typ II ausgelöst, das sich nach Standardmethoden von Miller und Rhodes (Meth. Enzymol. 82 (1982) S. 33) aus der Nasenscheidewand des Kalbes gewinnen ließ und den Tieren im Gemisch mit inkomplettem Freundschem Adjuvans intradermal injiziert wurde. Dieser Immunisierungsvorgang wurde 7 Tage später wiederholt. 20 Tage nach der Erstimmunisierung teilte man die erkrankten Ratten in Gruppen zu jeweils 7 Tieren auf, die in der anschließenden 20-tägigen Behandlungsphase einmal täglich die jeweilige Prüfsubstanz bzw. das reine Vehikel (Kontrollgruppe) oral erhielten. Am 41. Versuchstag, also einen Tag nach der letzten Substanzgabe, wurde die Volumenzunahme beider Hinterpfoten bestimmt.
Hierbei zeigte etwa die Verbindung des Beispiels 1 eine dosisabhängige Hemmung der Pfotenvolumenzunahme, die ab Dosen von 25 mg/kg p. o. statistisch signifikant war, während sich für Naproxen in gleicher Dosierung nur nicht-signifikante Hemmwerte ergaben.

Auch in diesem Modell der Collagen-Arthritis ist der Immunstatus der Lymphocyten empfindlich gestört. Daher wurden aus der Milz der Versuchstiere Lymphocyten gewonnen und deren Immunaktivität gegenüber Mitogenen untersucht, wobei sich wiederum für die erfindungsgemäßen Verbindungen dosisabhängige kurative Effekte auf das stark geschwächte Immunsystem nachweisen ließen, während Naproxen keine Wirkung entfaltete. So wurde beispielsweise mit einer Dosis von 12 mg/kg p. o. der Verbindung aus Beispiel 1 die Immunfunktion sowohl der T- als auch der B-Lymphocyten vollständig normalisiert.

c) Aktive Arthus-Reaktion

Als Versuchstiere dienten weibliche und männliche Sprague-Dawley Ratten mit einem Körpergewicht zwischen 80 und 100 g, denen 0,5 ml einer Emulsion von Pertussis-Vakzine und Ovalbumin in Paraffinöl subkutan in die Schwanzwurzel injiziert wurde. Nach zwei Wochen wurden die Ratten in Gruppen von jeweils 8 Tieren aufgeteilt. 24 Stunden und eine Stunde vor Auslösung der Arthus-Reaktion durch Injektion von 0,1 ml einer 0,4 %igen Ovalbumin-Lösung in die rechte Hinterpfote erfolgte die orale Verabreichung der jeweiligen Prüfsubstanz oder des reinen Vehikels (Positivkontrolle). In die linke Pfote wurde Natrium= chlorid-Lösung injiziert. Eine Gruppe nicht-sensibilisierter Tiere (Negativkontrolle) wurde gleichfalls mit Ovalbumin behandelt, um unspezifische Reaktionen gegenüber dem Protein ausschließen zu können. Als Meßparameter für die Präparatewirkung diente die Hemmung der Pfotenvolumenzunahme gegenüber jener der zwar sensibilisierten, aber unbehandelten Kontrollgruppe (Positivkontrolle) 4 Stunden nach der Ovalbumin-Provokation, wenn die Schwellung ihren Maximalwert erreicht.

Nicht-steroidale Antiphlogistika einschließlich Naproxen sind in dieser Versuchsanordnung wirkungslos. Demgegenüber ließ sich die Arthus-Reaktion nach oraler Verabreichung z. B. der Verbindung gemäß Beispiel 1 mit einer $ED_{50}$ zwischen 10 und 15 mg/kg eindrucksvoll hemmen.

5. Antioxydative Wirkung

Nach heutiger Lehrmeinung sind an dem multifaktoriell bedingten, progressiven Verlauf der rheumatoiden Arthritis und anderer entzündlicher Erkrankungen aggressive Sauerstoffradikale maßgeblich beteiligt, die während des chronischen Entzündungsprozesses exzessiv gebildet werden und selbst als hochtoxische Entzündungsmediatoren die über eine irreversible Lipidperoxydation der Zellmembranen verlaufende Bindegewebszerstörung perpetuell unterhalten. Folglich sollten antioxydativ wirksame Pharmaka mit der Fähigkeit zur Desaktivierung dieser extrem cytotoxischen Sauerstoffradikale einen gezielten Eingriff in das chronische Entzündungsgeschehen gestatten. Ein geeignetes Tiermodell für eine derartige durch Sauerstoffradikale vermittelte Gewebszerstörung stellt die an der Ratte mit Adriamycin (Doxorubicin) induzierte Entzündung dar.

a) Adriamycin-induzierte Entzündung

Die Untersuchungen wurden nach der Methode von D.M. Siegel et al. (Inflammation 4 (1980) S. 233) an männlichen Sprague-Dawley Ratten mit einem Körpergewicht zwischen 200 und 230 g in Gruppen von jeweils 7 Tieren durchgeführt, die 0,1 mg Adriamycin, gelöst in 0,1 ml 0,9%iger Kochsalzlösung, durch subkutane Injektion in die linke Hinterpfote erhielten. 72 Stunden danach wurde die Pfotenvolumenzunahme als Maß für den Entzündungsgrad durch plethysmographische Messung bestimmt.
Die orale Verabreichung der Prüfpräparate in 1%iger wäßriger Carboxymethylcellulose-Suspension erfolgte einmal täglich an 4 aufeinanderfolgenden Tagen, beginnend mit dem Tag der Adriamycin-Injektion.

Wie aus Tabelle 3 hervorgeht, zeigte auch in diesem Test beispielsweise die Verbindung aus Beispiel 1 einen starken, dosisabhängigen Schutzeffekt gegen die durch Adriamycin ausgelöste Gewebszerstörung. Sowohl steroidale als auch nichtsteroidale Antiphlogistika, einschließlich Naproxen, sind in dieser Versuchsanordnung unwirksam.

Tabelle 3: Hemmung der Adriamycin-induzierten Entzündung

| Tier-kollektiv | Dosis in mg/kg p.o. | Pfotenvolumen-zunahme in µl | Schutzwirkung in % |
|---|---|---|---|
| Kontrolle | - | 430 | - |
| Verbindung aus Beispiel 1 | 20 | 220 | 49* |
| | 40 | 200 | 54* |
| | 80 | 10 | 98* |

* Signifikanz $p < 0{,}05$

b) In-vitro-Hemmung der Lipidperoxydation

Einen weiteren überzeugenden Beleg für die ausgeprägte Schutzwirkung der erfindungsgemäßen Verbindungen gegenüber den aggressiven Sauerstoffradikalen lieferte der ThiobarbitursäureTest nach A. Ottolenghi (Arch. Biochem. Biophys. 79 (1959) S. 355-363). Mit dieser In-vitro-Methode läßt sich anhand des beim oxydativen Abbau membrangebundener, mehrfach ungesättigter Fettsäuren entstehenden Malondialdehyds die Beeinflussung sowohl der mirkosomalen als auch der mitochondrialen Lipidperoxydation durch antioxydativ wirksame Präparate bestimmen.

Auch hier übten die Verbindungen der Formel I eine starke Hemmwirkung aus. Für die Verbindung des Beispiels 1 ergaben sich mit Mikrosomen und Mitochondrien aus der Rattenleber biespielsweise $IC_{50}$-Werte von $6 \cdot 10^{-7}$ bzw. $3 \cdot 10^{-6}$ mol/l.

6. Hemmung der 5-Lipoxygenase

Die Hemmwirkung der erfindungsgemäßen Verbindungen auf den durch die 5-Lipoxygenase katalysierten Arachidonsäure-Abbau wurde, wie üblich, in In-vitro-Versuchen an isolierten polymorphkernigen Humangranulozyten nachgewiesen. Hierzu wurden die durch den Calcium-Ionophor A 23 187 (Calbiochem GmbH, Frankfurt/Main, BRD, Biochemical and Immunochemical Catalog 1985, S. 284) stimulierten Zellen mit $^{14}C$-markierter Arachidonsäure inkubiert und die nach 15 Minuten bei 37 °C durch Biotransformation gebildeten radioaktiven Hauptabbauprodukte der Arachidonsäure, die 5-Hydroxyeicosatetraensäure (5-HETE) und das besonders stark proinflammatorisch wirkende Leukotrien $B_4$ ($LTB_4$), nach Auftrennung durch Hochdruckflüssigkeitschromatographie (HPLC) mit Hilfe eines Radio-Monitors quantitativ bestimmt.
In dieser Versuchsanordnung ließ sich die Bildung sowohl von $LTB_4$ als auch 5-HETE und demzufolge der Arachidonsäure-Abbau über die 5-Lipoxygenase durch 15-minütige Vorinkubation der Granulozyten etwa mit der Verbindung des Beispiels 1 im Konzentrationsbereich zwischen $10^{-5}$ und $10^{-6}$ mol/l signifikant hemmen.

**Patentansprüche**
**Patentansprüche für folgende Vertragsstaaten : AT, BE, CH, DE, FR, GB, IT, LI, LU, NL, SE**

1. Substituierte 3-Phenyl-7H-thiazolo[3,2-b][1,2,4]triazin-7-one der allgemeinen Formel I

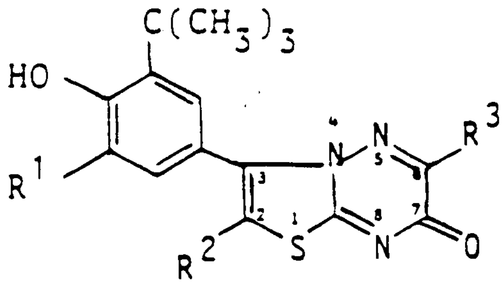

(I) ,

in der

| | |
|---|---|
| $R^1$ | eine geradkettige oder verzweigte Alkylgruppe mit 1 bis 4 C-Atomen, Hydroxymethyl oder eine Aminomethylgruppe der Formel II |

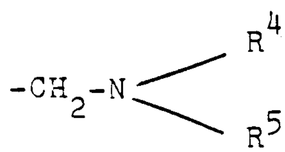

(II),

| | |
|---|---|
| $R^2$ | ein Wasserstoffatom oder einen Alkylrest mit 1 bis 3 C-Atomen und |
| $R^3$ | ein Wasserstoffatom, eine geradkettige oder verzweigte Alkylgruppe mit 1 bis 4 C-Atomen, Hydroxymethyl oder eine Aminomethylgruppe der Formel II bedeuten, wobei |
| $R^4$ und $R^5$ | gleich oder verschieden sind und ein Wasserstoffatom oder einen geradkettigen oder verzweigten Alkylrest mit 1 bis 4 C-Atomen darstellen, oder beide Reste zusammen mit dem Stickstoffatom, an das sie gebunden sind, einen 5- bis 7-gliedrigen gesättigten Ring mit 4 bis 6 C-Atomen oder mit 4 oder 5 C-Atomen und zusätzlich einem weiteren Heteroatom in Form von O, S oder $NR^6$ bilden und $R^6$ die Bedeutung von Wasserstoff oder $(C_1-C_4)$Alkyl hat, |

und die physiologisch verträglichen Säureadditionssalze der Verbindungen mit dem Strukturelement der Formel II in den Positionen von $R^1$ und/oder $R^3$.

2. Verbindungen nach Anspruch 1, gekennzeichnet durch wenigstens eines der Merkmale, daß $R^1$ einen tert.Butylrest oder eine Aminomethylgruppe der Formel II bedeutet und daß $R^2$ und $R^3$ unabhängig voneinander für Wasserstoff oder Methyl stehen.

3. Verbindungen nach Anspruch 1 oder 2, dadurch gekennzeichnet, daß $R^1$ eine tert.Butylgruppe darstellt und gleichzeitig $R^2$ und $R^3$ unabhängig voneinander für Wasserstoff oder Methyl stehen.

4. Verbindung nach Anspruch 3, dadurch gekennzeichnet, daß $R^1$ = tert.Butyl und $R^2$ und $R^3$ Wasserstoff bedeuten ( = 3-(3,5-Di-tert.butyl-4-hydroxyphenyl)-7H-thiazolo[3,2-b][1,2,4]triazin-7-on).

5. Verfahren zur Herstellung von substituierten 3-Phenyl-7H-thiazolo[3,2-b][1,2,4-]triazin-7-onen der Formel I gemäß Anspruch 1, wobei
$R^1$ eine geradkettige oder verzweigte Alkylgruppe mit 1 bis 4 C-Atomen bedeutet und
$R^2$ und $R^3$ die in Anspruch 1 genannten Bedeutungen haben, dadurch gekennzeichnet, daß man

a) ein 3-Mercapto-2H-1,2,4-triazin-5-on der Formel III

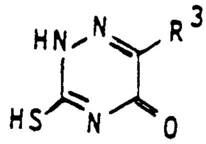

(III)

mit einem 2-Halogen-1-phenylalkanon der Formel IV

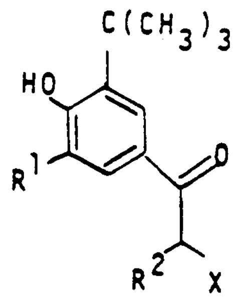

(IV)

wobei X für ein Halogenatom steht, zu einer Verbindung der Formel I umsetzt oder
b) eine Verbindung der Formel III mit einer Verbindung der Formel IV (mit der dort angegebenen Bedeutung der Reste $R^1$, $R^2$ und X) unter basischen Bedingungen zunächst zu einem S-alkylierten 2H-1,2,4-Triazin-5-on der Formel V

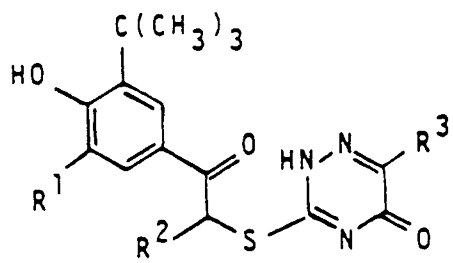

(V)

umsetzt und dieses unter Dehydratisierung in eine Verbindung der Formel I umwandelt.

**6.** Verfahren zur Herstellung von substituierten 3-Phenyl-7H-thiazolo[3,2-b][1,2,4-]triazin-7-onen der Formel I gemäß Anspruch 1, wobei

$R^1$ eine geradkettige oder verzweigte Alkylgruppe mit 1 bis 4 C-Atomen und
$R^3$ ein Wasserstoffatom, eine geradkettige oder verzweigte Alkylgruppe mit 1 bis 4 C-Atomen oder eine Hydroxymethylgruppe bedeutet und
$R^2$ die in Anspruch 1 genannte Bedeutung hat,

dadurch gekennzeichnet, daß man ein 2-Halogen-1-phenylalkanon der Formel IV (mit der dort angegebenen Bedeutung der Reste $R^1$, $R^2$ und X) zuerst mit Thiosemicarbazid zu dem entsprechenden 2-Amino-6H-1,3,4-thiadiazinder Formel VI

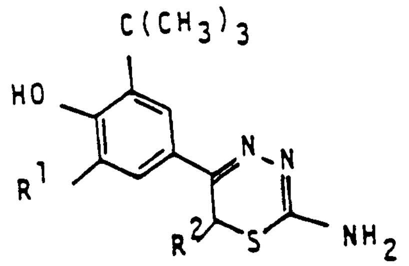

(VI)

umsetzt und dieses unter sauren Bedingungen in ein 3-Amino-2-imino-2,3-dihydro-thiazol der Formel VII

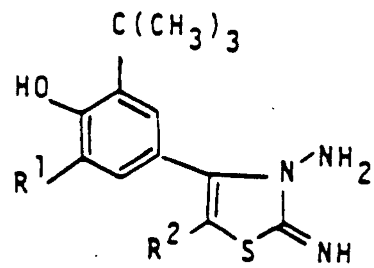

(VII)

umlagert, das anschließend mit einer α-Ketocarbonsäure bzw. deren Alkylestern der Formel VIII,

$R^3$-C-C-O-$R^7$ (VIII)
‖ ‖
O O

wobei $R^7$ ein Wasserstoffatom oder einen Alkylrest mit 1 bis 3 C-Atomen bedeutet, zu einer Verbindung der Formel I cyclokondensiert.

**7.** Verfahren zur Herstellung von substituierten 3-Phenyl-7H-thiazolo[3,2-b][1,2,4-]triazin-7-onen der Formel I gemäß Anspruch 1 und gegebenenfalls ihren physiologisch verträglichen Säureadditionssalzen in der $R^1$ und/oder $R^3$ die Bedeutung der Formel II hat, wobei $R^2$, $R^4$, $R^5$ die im Anspruch 1 genannten Bedeutungen haben, dadurch gekennzeichnet, daß man ausgehend von einer Verbindung der Formel I, die in der Position $R^1$ und/oder $R^3$ einen Hydroxymethyl-Rest trägt, zunächst deren Hydroxygruppe(n) gegen Halogen austauscht oder in einen aktivierten Sulfon- oder Phosphorsäureester überführt und anschließend mit einem Amin der Formel IX

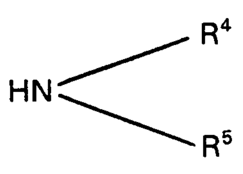

(IX)

zu einer Verbindung der Formel I umsetzt, und diese Aminomethyl-Verbindung entweder in freier Form isoliert oder aber mit geeigneten Säuren in physiologisch verträgliche Additionssalze umwandelt.

**8.** Verfahren zur Herstellung von substituierten 3-Phenyl-7H-thiazolo[3,2-b][1,2,4-]triazin-7-onen der Formel I gemäß Anspruch 1 und gegebenenfalls ihren physiologisch verträglichen Säureadditionssalzen in der $R^1$ Hydroxymethylrest oder Formel II bedeutet und $R^2$ und $R^3$ die in Anspruch 1 genannte Bedeutung haben, dadurch gekennzeichnet, daß man ausgehend von einer Verbindung der Formel I, in der $R^1$ für Wasserstoffatom steht und $R^2$ und $R^3$ die in Anspruch 1 genannte Bedeutung haben, diese zur

Einführung des Hydroxymethyl-Restes mit Formaldehyd umsetzt, zur Einführung von N-substituierten Aminomethylgruppen der Formel II mit den entsprechenden Aminen der Formel IX in Gegenwart von Formaldehyd umsetzt oder zur Einführung des unsubstituierten Aminomethyl-Restes zunächst mit N-Hydroxymethylacetamiden der Formel X,

$R^8$-CO-NH-$CH_2$-OH (X)

in der $R^8$ für Trifluormethyl, Trichlormethyl oder Chlormethyl steht, unter sauren Bedingungen kondensiert und anschließend den Acylrest $R^8$-CO-hydrolytisch eliminiert, wobei $R^2$ bis $R^5$ die in Anspruch 1 genannten Bedeutungen haben, und die so erhaltenen Aminomethyl-Verbindungen entweder in freier Form isoliert oder mit geeigneten Säuren in physiologisch verträgliche Additionssalze überführt.

**9.** Verbindungen der Formel I und die physiologisch verträglichen Säureadditionssalze dieser Verbindungen mit dem Strukturelement der Formel II in Position $R^1$ und/oder $R^3$ gemäß einem oder mehreren der Ansprüche 1 - 4 oder wie erhalten nach den Verfahren gemäß Ansprüche 5 bis 8 zur Anwendung als Heilmittel.

**10.** Arzneimittel, gekennzeichnet durch einen Gehalt an - oder bestehend aus - mindestens einer Verbindung der Formel I und/oder gegebenenfalls mindestens einem ihrer physiologisch verträglichen Säureadditionssalze nach einem oder mehreren der Ansprüche 1 bis 4 oder mindestens einer nach den Verfahren gemäß Ansprüche 5 bis 8 hergestellten Verbindung.

**11.** Arzneimittel nach Anspruch 10 zur Vorbeugung und Behandlung von Krankheiten, bei denen die therapeutische Anwendung von Entzündungshemmern, Immunmodulatoren, Sauerstoffradikale desaktivierenden Mitteln und/oder Hemmstoffen des durch die 5-Lipoxygenase vermittelten Arachidonsäureabbaus indiziert ist.

**12.** Arzneimittel nach Anspruch 10 oder 11 zur Vorbeugung und Behandlung von entzündlichen, insbesondere von entzündlich rheumatischen Erkrankungen.

**13.** Verfahren zur Herstellung eines Arzneimittels gemäß einem oder mehreren der Ansprüche 10 bis 12, dadurch gekennzeichnet, daß man mindestens eine Verbindung der Formel I und/oder mindestens ein physiologisch verträgliches Säureadditionssalz einer solchen Verbindung mit dem Strukturelement der Formel II in Position $R^1$ und/oder $R^3$ und/oder mindestens eine der nach den Verfahren gemäß Ansprüche 5 bis 8 erhaltenen Verbindungen mit einem physiologisch annehmbaren Träger und gegebenenfalls weiteren Zusatz- und/oder Hilfsstoffen in eine geeignete Darreichungsform bringt.

**14.** Verwendung von Verbindungen der Formel I und/oder gegebenenfalls deren physiologisch verträglichen Säureadditionssalzen zur Herstellung von Arzneimitteln gemäß den Ansprüchen 11 und 12.

**15.** S-alkylierte 2H-1,2,4-Triazin-5-one der Formel V

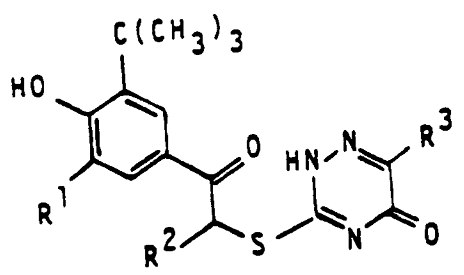

(V)

in der $R^1$ $(C_1-C_4)$-Alkyl bedeutet und $R^2$ und $R^3$ die gleiche Bedeutung wie in Formel I gemäß Anspruch 1 besitzen.

**16.** Verfahren zur Herstellung von S-alkylierten 2H-1,2,4-Triazin-5-onen der Formel V gemäß Anspruch 12, dadurch gekennzeichnet, daß man 3-Mercapto-2H-1,2,4-triazin-5-one der Formel III

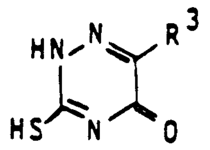

(III)

worin $R^3$ die gleiche Bedeutung wie in Formel I besitzt, mit 2-Halogen-1-phenylalkanonen der Formel IV

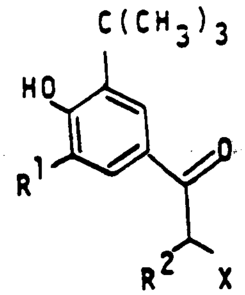

(IV)

worin $R^1$ $(C_1-C_4)$Alkyl bedeutet und $R^2$ die gleiche Bedeutung wie in Formel I besitzt und X = Halogen ist, unter basischen Bedingungen zur Reaktion bringt.

**Patentansprüche für folgende Vertragsstaaten : ES, GR**

**1.** Verfahren zur Herstellung von substituierten 3-Phenyl-7H-thiazolo[3,2-b][1,2,4]triazin-7-onen der allgemeinen Formel I

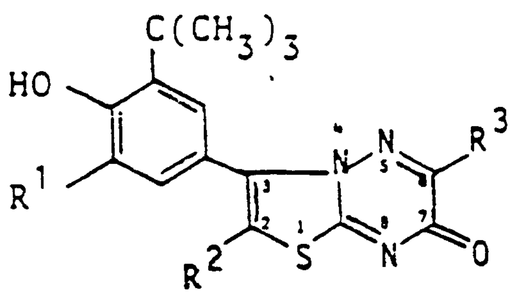

(I) ,

in der

$R^1$ eine geradkettige oder verzweigte Alkylgruppe mit 1 bis 4 C-Atomen, Hydroxymethyl oder eine Aminomethylgruppe der Formel II

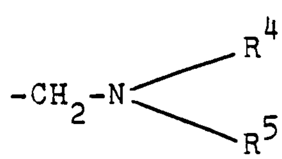

(II),

$R^2$ ein Wasserstoffatom oder einen Alkylrest mit 1 bis 3 C-Atomen und

$R^3$ ein Wasserstoffatom, eine geradkettige oder verzweigte Alkylgruppe mit 1 bis 4 C-Atomen, Hydroxymethyl oder eine Aminomethylgruppe der Formel II bedeuten, wobei

$R^4$ und $R^5$ gleich oder verschieden sind und ein Wasserstoffatom oder einen geradkettigen oder verzweigten Alkylrest mit 1 bis 4 C-Atomen darstellen, oder beide Reste zusammen mit dem Stickstoffatom, an das sie gebunden sind, einen 5- bis 7-gliedrigen gesättigten Ring mit 4 bis 6 C-Atomen oder mit 4 oder 5 C-Atomen und zusätzlich einem weiteren Heteroatom in Form von O, S oder $NR^6$ bilden und $R^6$ die Bedeutung von Wasserstoff oder $(C_1-C_4)$Alkyl hat,

und der physiologisch verträglichen Säureadditionssalze der Verbindungen mit dem Strukturelement der Formel II in den Positionen von $R^1$ und oder $R^3$,

dadurch gekennzeichnet, daß man

a) ein 3-Mercapto-2H-1,2,4-triazin-5-on der Formel III

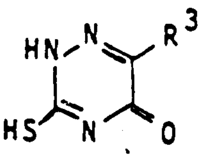

(III)

mit einem 2-Halogen-1-phenylalkanon der Formel IV

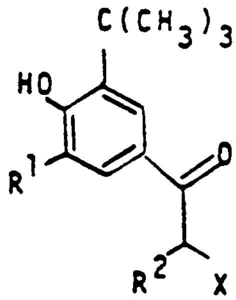

(IV)

zu einer Verbindung der Formel I umsetzt, wobei $R^1$ die im Anspruch 1 definierte $(C_1-C_4)$-Alkylgruppe darstellt und $R^2$ und $R^3$ die im Anspruch 1 genannten Bedeutungen haben und X für ein Halogenatom steht, oder

b) eine Verbindung der Formel III mit einer Verbindung der Formel IV (mit der dort angegebenen Bedeutung der Reste $R^1$, $R^2$ und X) unter basischen Bedingungen zunächst zu einem S-alkylierten 2H-1,2,4-Triazin-5-on der Formel V

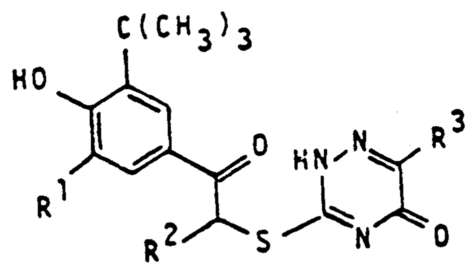

(V)

umsetzt und dieses unter Dehydratisierung in eine Verbindung der Formel I umwandelt, wobei $R^1$ die im Anspruch 1 definierte $(C_1-C_4)$Alkylgruppe darstellt und $R^2$ und $R^3$ die bei Formel I genannten Bedeutungen haben, oder

c) ein 2-Halogen-1-phenylalkanon der Formel IV (mit der dort angegebenen Bedeutung der Reste $R^1$, $R^2$ und X) zuerst mit Thiosemicarbazid zu dem entsprechenden 2-Amino-6H-1,3,4-thiadiazin der Formel VI

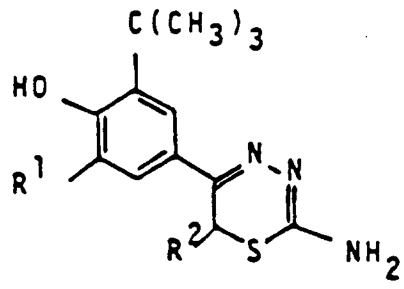

(VI)

umsetzt und dieses unter sauren Bedingungen in ein 3-Amino-2-imino-2,3-dihydro-thiazol der Formel VII

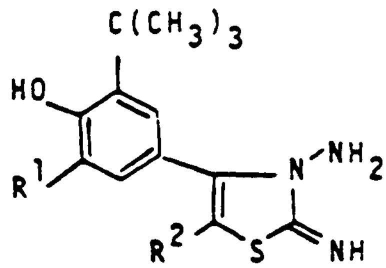

(VII)

umlagert, das anschließend mit einer α-Ketocarbonsäure bzw. deren Alkylestern der Formel VIII,

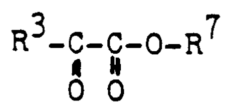

(VIII)

zu einer Verbindung der Formel I cyclokondensiert wird, wobei $R^1$ die im Anspruch 1 definierte ($C_1$-$C_4$)Alkylgruppe darstellt, $R^2$ die bei Formel I genannten Bedeutungen hat, $R^3$ ein Wasserstoffatom, eine geradkettige oder verzweigte Alkylgruppe mit 1 bis 4 C-Atomen oder eine Hydroxymethylgruppe bedeutet und $R^7$ ein Wasserstoffatom oder einen Alkylrest mit 1 bis 3 C-Atomen darstellt, oder daß man

d) ausgehend von einer Verbindung der Formel I, die in der Position $R^1$ und/oder $R^3$ einen Hydroxymethyl-Rest trägt, zunächst deren Hydroxygruppe(n) gegen Halogen austauscht oder in einen aktivierten Sulfon- oder Phosphorsäureester überführt und anschließend mit einem Amin der Formel IX

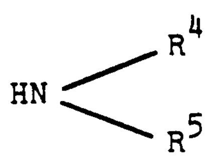

(IX)

zu einer Verbindung der Formel I mit dem Strukturelement der Formel II in der Stellung von $R^1$ und/oder $R^3$ umsetzt, wobei $R^2$, $R^4$, $R^5$ und gegebenenfalls auch $R^1$ bzw. $R^3$ die im Anspruch 1 genannten Bedeutungen haben, und diese Aminomethyl-Verbindung entweder in freier Form isoliert oder aber mit geeigneten Säuren in physiologisch verträgliche Additionssalze umwandelt, oder daß man

e) zur Herstellung einer Verbindung der Formel I mit dem Strukturmerkmal der Formel II oder dem Hydroxymethylrest in der Position von $R^1$ von einer Verbindung der Formel I, in der $R^1$ für Wasserstoff steht, ausgeht und diese zur Einführung des Hydroxymethyl-Restes mit Formaldehyd umsetzt, zur Einführung von N-substituierten Aminomethylgruppen der Formel II mit den entsprechenden Aminen der Formel IX in Gegenwart von Formaldehyd umsetzt oder zur Einführung des unsubstituierten Aminomethyl-Restes zunächst mit N-Hydroxymethylacetamiden der Formel X,

$R^8$-CO-NH-$CH_2$-OH (X)

in der $R^8$ für Trifluormethyl, Trichlormethyl oder Chlormethyl steht, unter sauren Bedingungen kondensiert und anschließend den Acylrest $R^8$-CO-hydrolytisch eliminiert, wobei $R^2$ bis $R^5$ die in Anspruch 1 genannten Bedeutungen haben, und die so erhaltenen Aminomethyl-Verbindungen entweder in freier Form isoliert oder mit geeigneten Säuren in physiologisch verträgliche Additionssalze überführt.

**2.** Verfahren nach Anspruch 1, dadurch gekennzeichnet, daß man Verbindungen der Formel I herstellt mit wenistens einem der Merkmale, daß $R^1$ einen tert.-Butylrest oder eine Aminomethylgruppe der Formel II darstellt und daß $R^2$ und $R^3$ unabhängig voneinander für Wasserstoff oder Methyl stehen.

**3.** Verfahren nach Anspruch 1 oder 2, dadurch gekennzeichnet, daß man Verbindungen der Formel I herstellt, worin $R^1$ eine tert.-Butylgruppe darstellt und gleichzeitig $R^2$ und $R^3$ unabhängig voneinander für Wasserstoff oder Methyl stehen.

**4.** Verfahren nach Anspruch 3, dadurch gekennzeichnet, daß man die Verbindung der Formel I mit $R^1$ = tert.-Butylgruppe und $R^2$ = $R^3$ = Wasserstoff herstellt (= 3-(3,5-Di-tert.butyl-4-hydroxyphenyl)-7H-thiazolo[3,2b][1,2,4]triazin-7-on).

**5.** Verfahren Zur Herstellung der Verbindungen der Formel I und der physiologisch verträglichen Säureadditionssalze dieser Verbindungen mit dem Strukturelement der Formel II in Position $R^1$ und/oder $R^3$ gemäß einem oder mehreren der Ansprüche 1 - u zur Anwendung als Heilmittel.

**6.** Verfahren zur Herstellung eines Arzneimittels, dadurch gekennzeichnet, daß man mindestens eine Verbindung der Formel I gemäß der Definition in Anspruch 1 und/oder mindestens ein physiologisch verträgliches Säureadditionssalz einer solchen Verbindung mit dem Strukturelement der Formel II in Position $R^1$ und/oder $R^3$ und/oder mindestens eine der nach dem Verfahren gemäß einem oder mehreren der Ansprüche 1 - 4 erhaltenen Verbindungen mit einem physiologisch annehmbaren Träger und gegebenenfalls weiteren Zusatz- und/oder Hilfsstoffen in eine geeignete Darreichungsform bringt.

**7.** Verwendung von Verbindungen der Formel I gemäß der Definition in Anspruch 1 oder wie erhalten nach einem oder mehreren der Ansprüche 1 - 4 und/oder gegebenenfalls von deren physiologisch verträglichen Säureadditionssalzen zur Herstellung von Arzneimitteln zur Vorbeugung und Behandlung von Krankheiten, bei denen die therapeutische Anwendung von Entzündungshemmer, Immunmodulatoren, Sauerstoffradikale desaktivierenden Mitteln und/oder Hemmstoffen des durch die 5-Lipoxygenase vermittelten Arachidonsäureabbaus indiziert ist oder zur Vorbeugung und Behandlung von entzündlichen, insbesondere von entzündlich rheumatischen Erkrankungen.

**8.** Verbindungen der Formel I und die physiologisch verträglichen Säureadditionssalze der Verbindungen der Formel I mit dem Strukturelement der Formel II in Position $R^1$ und/oder $R^3$, hergestellt nach dem Verfahren gemäß einem oder mehreren der Ansprüche 1 - 4.

**9.** Arzneimittel, hergestellt nach dem Verfahren gemäß Anspruch 6.

**10.** S-alkyliert 2H-1,2,4-Triazin-5-one der Formel V

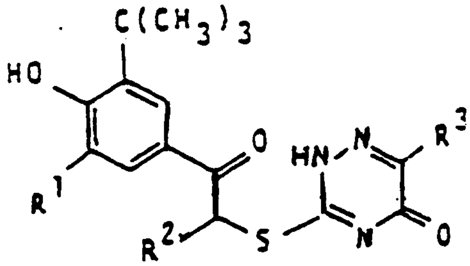

(V)

in der $R^1$ $(C_1-C_4)$-Alkyl bedeutet und $R^2$ und $R^3$ die gleiche Bedeutung wie in Formel I gemäß Anspruch 1 besitzen.

**11.** Verfahren zur Herstellung von S-alkylierten 2H-1,2,4-Triazin-5-onen der Formel V gemäß Anspruch 12, dadurch gekennzeichnet, daß man 3-Mercapto-2H-1,2,4-triazin-5-one der Formel III

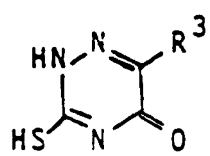

(III)

worin $R^3$ die gleiche Bedeutung wie in Formel I besitzt, mit 2-Halogen-1-phenylalkanonen der Formel IV

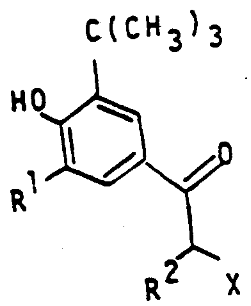

(IV)

worin $R^1$ $(C_1-C_4)$Alkyl bedeutet und $R^2$ die gleiche Bedeutung wie in Formel I besitzt und X = Halogen ist, unter basischen Bedingungen zur Reaktion bringt.

**Claims**

**Claims for the following Contracting States : AT, BE, CH, DE, FR, GB, IT, LI, LU, NL, SE**

**1.** A substituted 3-phenyl-7H-thiazolo[3,2-b][1,2,4]triazin-7-one of the formula I

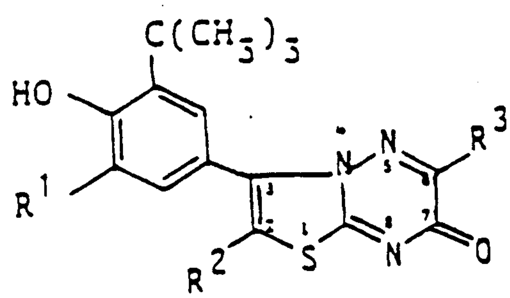

(I)

in which

$R^1$ is a straight-chain or branched alkyl group having 1 to 4 carbon atoms, hydroxymethyl or an aminomethyl group of the formula II

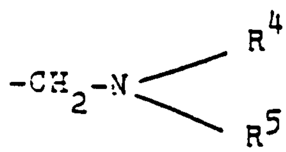

(II)

$R^2$ is a hydrogen atom or an alkyl radical having 1 to 3 carbon atoms, and

$R^3$ is a hydrogen atom, a straight-chain or branched alkyl group having 1 to 4 carbon atoms, hydroxymethyl or an aminomethyl group of the formula II,

where

$R^4$ and $R^5$ are identical or different and are a hydrogen atom or a straight-chain or branched alkyl radical having 1 to 4 carbon atoms, or the two radicals, together with the nitrogen atom to which they are bound, form a 5- to 7-membered saturated ring having 4 to 6 carbon atoms or having 4 or 5 carbon atoms and additionally having a further heteroatom in the form of O, S or $NR^6$, and $R^6$ is hydrogen or $(C_1-C_4)$alkyl,

or a physiologically acceptable acid-addition salt of the compound having the structural element of the formula II in the positions of $R^1$ and/or $R^3$.

**2.** A compound as claimed in claim 1, comprising at least one of the features that $R^1$ is a tert-butyl radical or an aminomethyl group of the formula II and that $R^2$ and $R^3$, independently of one another, are hydrogen or methyl.

**3.** A compound as claimed in claim 1 or 2, wherein $R^1$ is a tert-butyl group and simultaneously $R^2$ and $R^3$, independently of one another, are hydrogen or methyl.

**4.** A compound as claimed in claim 3, wherein $R^1$ = tert-butyl and $R^2$ and $R^3$ are hydrogen (= 3-(3,5-di-tert-butyl-4-hydroxyphenyl)-7H-thiazolo[3,2-b][1,2,4]triazin-7-one).

**5.** A process for the preparation of a substituted 3-phenyl-7H-thiazolo[3,2-b][1,2,4]triazin-7-one of the formula I as claimed in claim 1, where

$R^1$ is a straight-chain or branched alkyl group having 1 to 4 carbon atoms and

$R^2$ and $R^3$ have the meanings mentioned in claim 1, which comprises

a) reacting a 3-mercapto-2H-1,2,4-triazin-5-one of the formula III

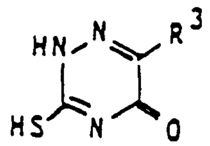

(III)

with a 2-halo-1-phenylalkanone of the formula IV

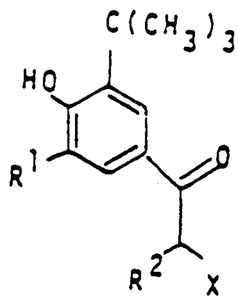

(IV)

where X is a halogen atom, to give a compound of the formula I, or

b) reacting a compound of the formula III with a compound of the formula IV (with the abovementioned meaning for the radicals $R^1$, $R^2$ and X) under basic conditions to give, initially, an S-alkylated 2H-1,2,4-triazin-5-one of the formula V

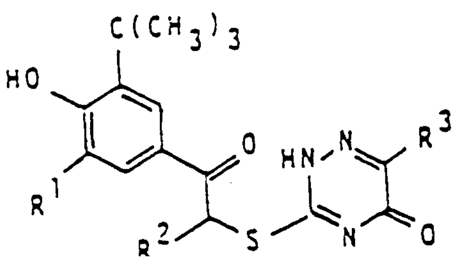

(V)

and converting the latter, with dehydration, into a compound of the formula I.

**6.** A process for the preparation of a substituted 3-phenyl-7H-thiazolo[3,2-b][1,2,4]triazin-7-one of the formula I as claimed in claim 1, where

$R^1$ is a straight-chain or branched alkyl group having 1 to 4 carbon atoms and

$R^3$ is a hydrogen atom, a straight-chain or branched alkyl group having 1 to 4 carbon atoms or a hydroxymethyl group and

$R^2$ has the meaning mentioned in claim 1,

which comprises reacting a 2-halo-1-phenylalkanone of the formula IV (with the abovementioned meaning for the radicals $R^1$, $R^2$ and X) initially with thiosemicarbazide to give the corresponding 2-amino-6H-1,3,4-thiadiazine of the formula VI

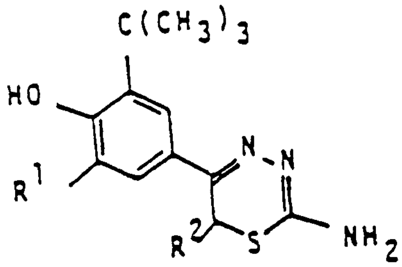

(VI)

and rearranging the latter under acidic conditions to give a 3-amino-2-imino-2,3-dihydrothiazole of the formula VII

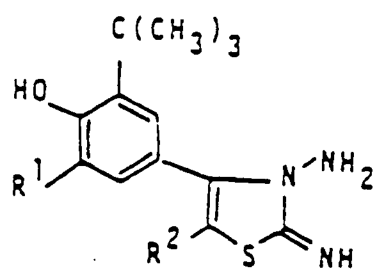

(VII)

which is subsequently cyclocondensed with an α-keto-carboxylic acid, or an alkyl ester thereof, of the formula VIII,

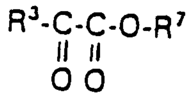

(VIII)

where $R^7$ is a hydrogen atom or an alkyl radical having 1 to 3 carbon atoms, to give a compound of the formula I.

**7.** A process for the preparation of a substituted 3-phenyl-7H-thiazolo[3,2-b][1,2,4]triazin-7-one of the formula I as claimed in claim 1 or, if desired, a physiologically acceptable acid-addition salt thereof, in which $R^1$ and/or $R^3$ has the meaning of formula II, where $R^2$, $R^4$, $R^5$ have the meanings mentioned in claim 1, which comprises starting from a compound of the formula I which carries a hydroxymethyl radical in the position of $R^1$ and/or $R^3$, initially either replacing the hydroxyl group(s) of said compound by halogen or converting the hydroxyl group(s) into an activated sulfonate or phosphate, and subsequently reacting the product of this reaction with an amine of the formula IX

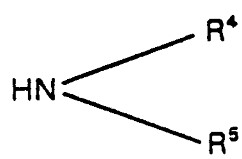

(IX)

to give a compound of the formula I, and either isolating this aminomethyl compound in free form or converting it into a physiologically acceptable addition salt by means of a suitable acid.

**8.** A process for the preparation of a substituted 3-phenyl-7H-thiazolo[3,2-b][1,2,4]triazin-7-one of the formula I as claimed in claim 1 or, if desired, a physiologically acceptable acid-addition salt thereof, in

which $R^1$ is a hydroxymethyl radical or formula II and $R^2$ and $R^3$ have the meaning mentioned in claim 1, which comprises starting from a compound of the formula I in which $R^1$ is a hydrogen atom and $R^2$ and $R^3$ have the meaning stated in claim 1, and reacting said compound with formaldehyde in order to introduce the hydroxymethyl radical, reacting said compound with the appropriate amines of the formula IX, in the presence of formaldehyde, in order to introduce N-substituted aminomethyl groups of the formula II or, in order to introduce the unsubstituted aminomethyl radical, initially condensing said compound with N-hydroxymethylacetamides of the formula X

$R^8$-CO-NH-$CH_2$-OH (X)

in which $R^8$ is trifluoromethyl, trichloromethyl or chloromethyl, under acidic conditions and subsequently eliminating the acyl radical $R^8$-CO- hydrolytically, $R^2$ to $R^5$ having the meanings mentioned in claim 1, and either isolating the resultant aminomethyl compound in free form or converting it into a physiologically acceptable addition salt by means of a suitable acid.

**9.** A compound of the formula I or a physiologically acceptable acid-addition salt of the compound having the structural element of the formula II in the position of $R^1$ and/or $R^3$, as claimed in one or more of claims 1 - 4 or as obtained by the process as claimed in any one of claims 5 to 8 for use as a medicament.

**10.** A medicament, which contains, or comprises, at least one compound of the formula I and/or, if desired, at least one physiologically acceptable acid-addition salt thereof as claimed in one or more of claims 1 to 4 or at least one compound prepared by the process as claimed in any one of claims 5 to 8.

**11.** A medicament as claimed in claim 10, for the prevention and treatment of disorders in which the therapeutic use of inflammation inhibitors, immune modulators, oxygen radical-deactivating agents and/or inhibitors of 5-lipoxygenase-induced degradation of arachidonic acid is indicated.

**12.** A medicament as claimed in claim 10 or 11 for the prevention and treatment of inflammatory disorders, in particular inflammatory rheumatic disorders.

**13.** A process for the production of a medicament as claimed in one or more of claims 10 to 12, wherein at least one compound of the formula I and/or at least one physiologically acceptable acid-addition salt of such a compound having the structural element of the formula II in the position of $R^1$ and/or $R^3$ and/or at least one compound obtained by the process as claimed in any one of claims 5 to 8 is converted into a suitable form of administration together with a physiologically acceptable excipient and, if appropriate, further additives and/or adjuvants.

**14.** The use of a compound of the formula I and/or, if desired, a physiologically acceptable acid-addition salt thereof for the preparation of a medicament as claimed in claims 11 and 12.

**15.** An S-alkylated 2H-1,2,4-triazin-5-one of the formula V

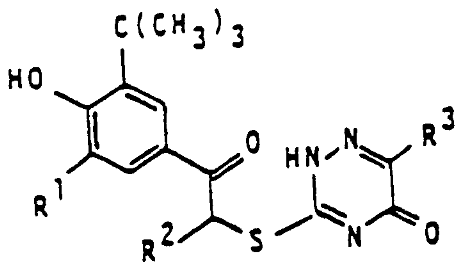

(V)

in which $R^1$ is $(C_1$-$C_4)$alkyl and $R^2$ and $R^3$ have the same meaning as in the formula I of claim 1.

**16.** A process for the preparation of an S-alkylated 2H-1,2,4-triazin-5-one of the formula V as claimed in claim 12, wherein a 3-mercapto-2H-1,2,4-triazin-5-one of the formula III

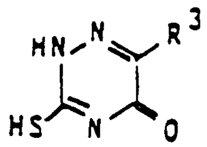

(III)

in which $R^3$ has the same meaning as in the formula I, is reacted with a 2-halo-1-phenylalkanone of the formula IV

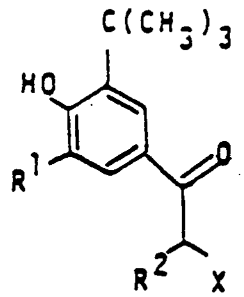

(IV)

in which $R^1$ is $(C_1\text{-}C_4)$alkyl and $R^2$ has the same meaning as in the formula I and X is halogen, under basic conditions.

**Claims for the following Contracting States : ES, GR**

**1.** A process for the preparation of a substituted 3-phenyl-7H-thiazolo[3,2-b][1,2,4]triazin-7-one of the formula I

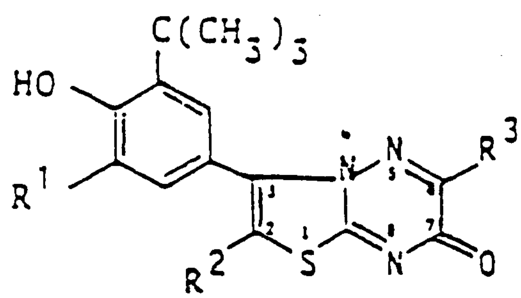

(I)

in which

| | |
|---|---|
| $R^1$ | is a straight-chain or branched alkyl group having 1 to 4 carbon atoms, hydroxymethyl or an aminomethyl group of the formula II |

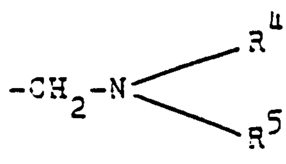

(II)

| | |
|---|---|
| $R^2$ | is a hydrogen atom or an alkyl radical having 1 to 3 carbon atoms, and |
| $R^3$ | is a hydrogen atom, a straight-chain or branched alkyl group having 1 to 4 carbon atoms, hydroxymethyl or an aminomethyl group of the formula II, where |
| $R^4$ and $R^5$ | are identical or different and are a hydrogen atom or a straight-chain or branched alkyl radical having 1 to 4 carbon atoms, or the two radicals, together with the nitrogen atom to which they are bound, form a 5- to 7-membered saturated ring |

having 4 to 6 carbon atoms or having 4 or 5 carbon atoms and additionally having a further heteroatom in the form of O, S or $NR^6$, and $R^6$ is hydrogen or $(C_1-C_4)$alkyl,

or a physiologically acceptable acid-addition salt of the compound having the structural element of the formula II in the positions of $R^1$ and/or $R^3$, which comprises

a) reacting a 3-mercapto-2H-1,2,4-triazin-5-one of the formula III

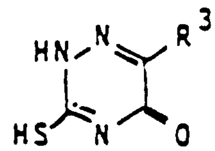

(III)

with a 2-halo-1-phenylalkanone of the formula IV

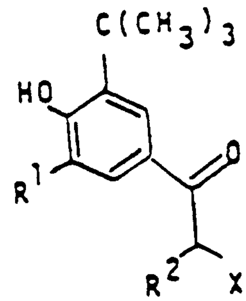

(IV)

to give a compound of the formula I where $R^1$ is the $(C_1-C_4)$alkyl group defined in claim 1 and $R^2$ and $R^3$ have the meanings mentioned in claim 1, and X is a halogen atom, or

b) reacting a compound of the formula III with a compound of the formula IV (with the abovementioned meaning for the radicals $R^1$, $R^2$ and X) under basic conditions to give, initially, an S-alkylated 2H-1,2,4-triazin-5-one of the formula V

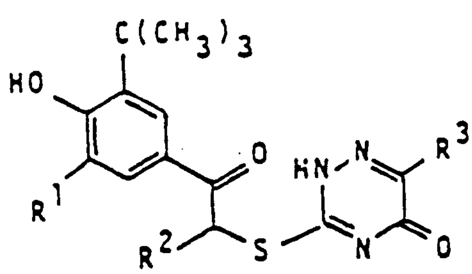

(V)

and converting the latter, with dehydration, into a compound of the formula I where $R^1$ is the $(C_1-C_4)$-alkylgroup defined in claim 1 and $R^2$ and $R^3$ have the meanings mentioned in formula 1, or

c) reacting a 2-halo-1-phenylalkanone of the formula IV (with the abovementioned meaning for the radicals $R^1$, $R^2$ and X) initially with thiosemicarbazide to give the corresponding 2-amino-6H-1,3,4-thiadiazine of the formula VI

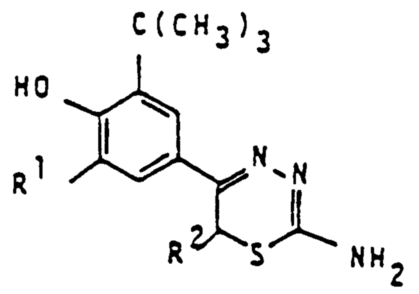

(VI)

and rearranging the latter under acidic conditions to give a 3-amino-2-imino-2,3-dihydrothiazole of the formula VII

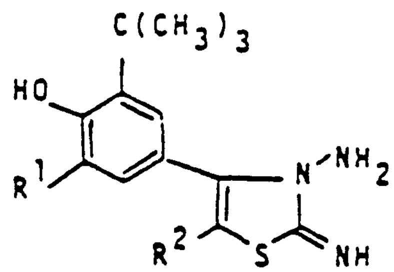

(VII)

which is subsequently cyclocondensed with an α-keto-carboxylic acid, or an alkyl ester thereof, of the formula VIII,

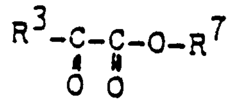

(VIII)

to give a compound of the formula I where $R^1$ is the ($C_1$-$C_4$)alkyl group defined in claim 1, $R^2$ has the meanings mentioned in formula 1, $R^3$ is a hydrogen atom, a straight-chain or branched alkyl group having 1 to 4 carbon atoms or a hydroxymethyl group, and $R^7$ is a hydrogen atom or an alkyl radical having 1 to 3 carbon atoms, or which comprises

d) starting from a compound of the formula I which carries a hydroxymethyl radical in the position of $R^1$ and/or $R^3$, initially either replacing the hydroxyl group(s) of said compound by halogen or converting the hydroxyl group(s) into an activated sulfonate or phosphate, and subsequently reacting the product of this reaction with an amine of the formula IX

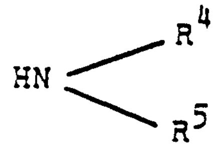

(IX)

to give a compound of the formula I having the structural element of the formula II in the position of $R^1$ and/or $R^3$, where $R^2$, $R^4$, $R^5$ and, if appropriate, also $R^1$ and $R^3$ have the meanings mentioned in claim 1, and either isolating this aminomethyl compound in free form or converting it into a physiologically acceptable addition salt by means of a suitable acid, or which comprises

e) for the preparation of a compound of the formula I having the structural feature of the formula II or the hydroxymethyl radical in the position of $R^1$, starting from a compound of the formula I in which $R^1$ is hydrogen and reacting said compound with formaldehyde in order to introduce the hydroxymethyl radical, reacting said compound with the appropriate amines of the formula IX, in the presence of formaldehyde, in order to introduce N-substituted aminomethyl groups of the formula II or, in order to introduce the unsubstituted aminomethyl radical, initially condensing said compound with N-hydroxymethylacetamides of the formula X

$R^8$-CO-NH-$CH_2$-OH (X)

in which $R^8$ is trifluoromethyl, trichloromethyl or chloromethyl, under acidic conditions and subsequently eliminating the acyl radical $R^8$-CO- hydrolytically, $R^2$ to $R^5$ having the meanings mentioned in claim 1, and either isolating the resultant aminomethyl compound in free form or converting it into a physiologically acceptable addition salt by means of a suitable acid.

**2.** The process as claimed in claim 1, wherein a compound of the formula I is prepared having at least one of the features that $R^1$ denotes a tert-butyl radical or an aminomethyl group of the formula II and that $R^2$ and $R^3$, independently of one another, are hydrogen or methyl.

**3.** The process as claimed in claim 1 or 2, wherein a compound of the formula I is prepared in which $R^1$ is a tert-butyl group and simultaneously $R^2$ and $R^3$, independently of one another, are hydrogen or methyl.

**4.** A compound as claimed in claim 3, wherein the compound of the formula I is prepared in which $R^1$ = a tert-butyl group and $R^2$ and $R^3$ are hydrogen (= 3-(3,5-di-tert-butyl-4-hydroxyphenyl)-7H-thiazolo[3,2-b]-[1,2,4]triazin-7-one).

**5.** A process for the preparation of a compound of the formula I or a physiologically acceptable acid-addition salt of such a compound having the structural element of the formula II in the position of $R^1$ and/or $R^3$, as claimed in one or more of claims 1 - 4 for use as a medicament.

**6.** A process for the production of a medicament, wherein at least one compound of the formula I as defined in claim 1 and/or at least one physiologically acceptable acid-addition salt of such a compound having the structural element of the formula II in the position of $R^1$ and/or $R^4$ and/or at least one compound obtained by the process as claimed in one or more of claims 1 - 4 is converted into a suitable form of administration together with a physiologically acceptable excipient and, if appropriate, further additives and/or adjuvants.

**7.** The use of a compound of the formula I as defined in claim 1 or obtained as claimed in one or more of claims 1 to 4 and/or, if appropriate, of a physiologically acceptable acid-addition salt thereof for the production of medicaments for the prevention and treatment of disorders in which the therapeutic use of inflammation inhibitors, immune modulators, oxygen radical-deactivating agents and/or inhibitors of 5-lipoxygenase-induced degradation of arachidonic acid is indicated or for the prevention and treatment of inflammatory disorders, in particular inflammatory rheumatic disorders.

**8.** A compound of the formula I or a physiologically acceptable acid-addition salt of a compound of the formula I having the structural element of the formula II in the position of $R^1$ and/or $R^3$, prepared by the process as claimed in one or more of claims 1 - 4.

**9.** A medicament, produced by the process as claimed in claim 6.

**10.** An S-alkylated 2H-1,2,4-triazin-5-one of the formula V

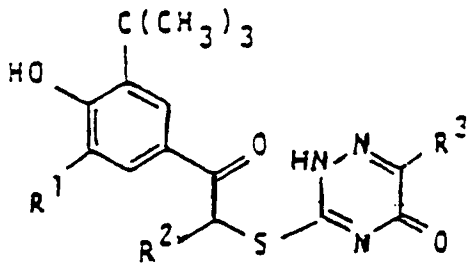

(V)

in which $R^1$ is $(C_1\text{-}C_4)$alkyl and $R^2$ and $R^3$ have the same meaning as in the formula I of claim 1.

**11.** A process for the preparation of an S-alkylated 2H-1,2,4-triazin-5-one of the formula V as claimed in claim 12, wherein a 3-mercapto-2H-1,2,4-triazin-5-one of the formula III

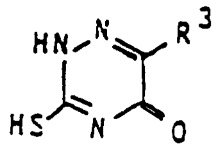

(III)

in which $R^3$ has the same meaning as in the formula I, is reacted with a 2-halo-1-phenylalkanone of the formula IV

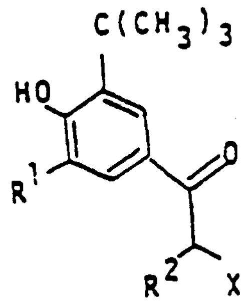

(IV)

in which $R^1$ is $(C_1\text{-}C_4)$alkyl and $R^2$ has the same meaning as in the formula I and X is halogen, under basic conditions.

**Revendications**

**Revendications pour les Etats contractants suivants : AT, BE, CH, DE, FR, GB, IT, LI, LU, NL, SE**

**1.** 3-Phényl-7H-thiazolo[3,2-b][1,2,4]triazine-7-ones substituées de formule générale I

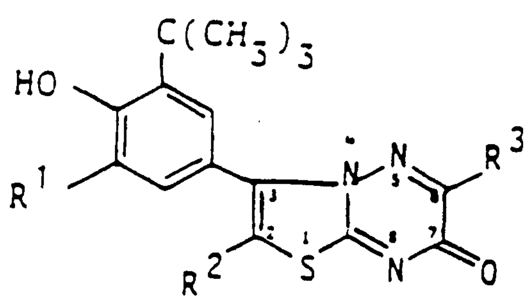

(I)

dans laquelle

$R^1$ représente un groupe alkyle à chaîne droite ou ramifiée ayant de 1 à 4 atomes de carbone, le groupe hydroxyméthyle ou un groupe aminométhyle de formule II

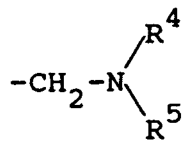

(II),

$R^2$ représente un atome d'hydrogène ou un radical alkyle ayant de 1 à 3 atomes de carbone, et

$R^3$ représente un atome d'hydrogène ou un groupe alkyle à chaîne droite ou ramifiée ayant de 1 à 4 atomes de carbone, le groupe hydroxyméthyle ou un groupe aminométhyle de formule II,

$R^4$ et $R^5$ étant identiques ou différents et représentant un atome d'hydrogène ou un radical alkyle à chaine droite ou ramifiée ayant de 1 à 4 atomes de carbone, ou les deux radicaux, conjointement avec l'atome d'azote auxquels ils sont liés, formant un cycle saturé à 5-7 chaînons ayant de 4 à 6 atomes de carbone ou ayant 4 ou 5 atomes de carbone et en outre un autre hétéroatome sous forme de O, S ou $NR^6$, et $R^6$ représentant un atome d'hydrogène ou un groupe alkyle en $C_1$-$C_4$,

et sels d'addition avec des acides physiologiquement acceptables des composés comportant l'élément structural de formule II en position(s) de $R^1$ et/ou $R^3$.

**2.** Composés selon la revendication 1, caractérisés par au moins l'une des caractéristiques que $R^1$ représente le radical tert-butyle ou un groupe aminométhyle de formule II et que $R^2$ et $R^3$, indépendamment l'un de l'autre, représentent un atome d'hydrogène ou le groupe méthyle.

**3.** Composés selon la revendication 1 ou 2, caractérisés en ce que $R^1$ représente le groupe tert-butyle et en même temps $R^2$ et $R^3$ représentent, indépendamment l'un de l'autre, un atome d'hydrogène ou le groupe méthyle.

**4.** Composé selon la revendication 3, caractérisé en ce que $R^1$ représente le groupe tert-butyle et $R^2$ et $R^3$ représentent des atomes d'hydrogène (= 3-(3,5-di-tert-butyl-4-hydroxyphényl)-7H-thiazolo[3,2-b]-[1,2,4]triazine-7-one).

**5.** Procédé pour la préparation de 3-phényl-7H-thiazolo[3,2-b][1,2,4]triazine-7-ones substituées de formule I selon la revendication 1, dans lesquelles

$R^1$ représente un groupe alkyle à chaîne droite ou ramifiée ayant de 1 à 4 atomes de carbone, et

$R^2$ et $R^3$ ont les significations données dans la revendication 1,

caractérisé en ce que

a) on fait réagir une 3-mercapto-2H-1,2,4-triazine-5-one de formule III

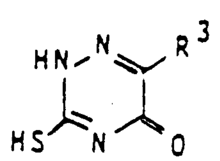

(III)

avec une 2-halogéno-1-phénylalcanone de formule IV

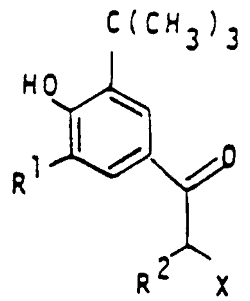

(IV)

X représentant un atome d'halogène, pour aboutir à un composé de formule I, ou

b) on fait réagir un composé de formule III avec un composé de formule IV (avec la signification donnée des radicaux $R^1$, $R^2$ et X) dans des conditions basiques, pour aboutir d'abord à une 2H-1,2,4-triazine-5-one S-alkylée de formule V

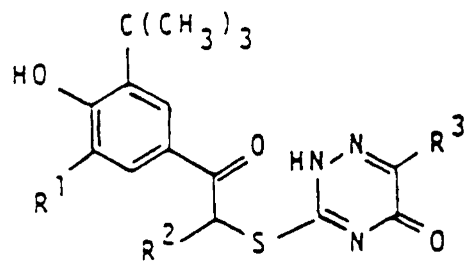

(V)

et on convertit celle-ci, par déshydratation, en un composé de formule I.

**6.** Procédé pour la préparation de 3-phényl-7H-thiazolo[3,2-b][1,2,4]triazine-7-ones substituées de formule I selon la revendication 1, dans lesquelles

$R^1$ représente un groupe alkyle à chaîne droite ou ramifiée ayant de 1 à 4 atomes de carbone et

$R^3$ représente un atome d'hydrogène ou un groupe alkyle à chaîne droite ou ramifiée ayant de 1 à 4 atomes de carbone, ou le groupe hydroxyméthyle, et

$R^2$ a la signification donnée dans la revendication 1,

caractérisé en ce que l'on fait réagir une 2-halogéno-1-phénylalcanone de formule IV (avec la signification des radicaux $R^1$, $R^2$ et X donnée dans celle-ci) en premier lieu avec du thiosemicarbazide, pour aboutir à la 2-amino-6H-1,3,4-thiadiazine correspondante de formule VI

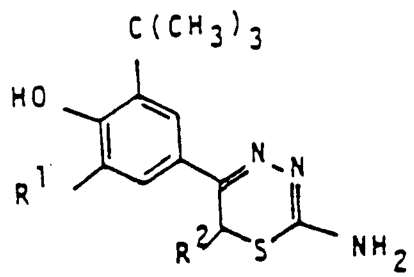

(VI)

et on convertit celle-ci par transposition, dans des conditions acides, en un 3-amino-2-imino-2,3-dihydro-thiazole de formule VII

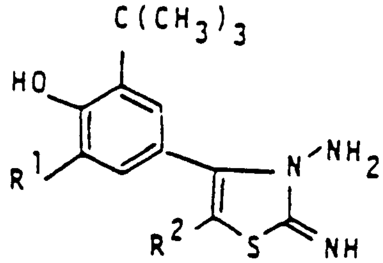

(VII)

qui est ensuite cyclocondensé avec un acide α-cétocarboxylique ou ses esters alkyliques de formule VIII

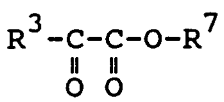

(VIII)

$R^7$ représentant un atome d'hydrogène ou un radical alkyle ayant de 1 à 3 atomes de carbone, pour donner un composé de formule I.

**7.** Procédé pour la préparation de 3-phényl-7H-thiazolo[3,2-b][1,2,4]triazine-7-ones substituées de formule I selon la revendication 1, et éventuellement de leurs sels d'addition avec des acides physiologiquement acceptables, dans lesquelles $R^1$ et/ou $R^3$ ont la signification de la formule II, $R^2$, $R^4$, $R^5$ ayant les significations données dans la revendication 1, caractérisé en ce que, à partir d'un composé de formule I qui porte en position(s) $R^1$ et/ou $R^3$ un radical hydroxyméthyle, en premier lieu on échange contre un halogène ou convertit en un ester sulfonique ou phosphorique activé son(ses) groupe(s) hydroxy et ensuite on le fait réagir avec une amine de formule IX

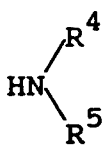

(IX)

pour aboutir à un composé de formule I, et ce composé aminométhyle est soit isolé sous forme libre, soit converti, avec des acides appropriés, en sels d'addition physiologiquement acceptables.

**8.** Procédé pour la préparation de 3-phényl-7H-thiazolo[3,2-b][1,2,4]triazine-7-ones substituées de formule I selon la revendication 1, et éventuellement de leurs sels d'addition avec des acides physiologiquement acceptables, dans lesquelles $R^1$ représente le radical hydroxyméthyle ou un groupe de formule II, et $R^2$ et $R^3$ ont les significations données dans la revendication 1, caractérisé en ce que, à partir d'un composé de formule I, dans lequel $R^1$ représente un atome d'hydrogène et $R^2$ et $R^3$ ont les significations données dans la revendication 1, pour l'introduction du radical hydroxyméthyle, on le fait réagir avec du formaldéhyde, pour l'introduction de groupes aminométhyle de formule II substitués à l'azote, on le fait réagir avec les amines de formule IX correspondantes, en présence de formaldéhyde, ou, pour l'introduction du radical aminométhyle non substitué, on le condense d'abord avec des hydroxyméthylacétamides de formule X

$R^8$-CO-NH-$CH_2$-OH (X)

dans laquelle $R^8$ représente le groupe trifluorométhyle, trichlorométhyle ou chlorométhyle, dans des conditions acides, et on élimine ensuite par hydrolyse le radical acyle $R^8$-CO-, $R^2$ à $R^5$ ayant les significations données dans la revendication 1, et les composés aminométhyle ainsi obtenus sont soit isolés sous forme libre, soit convertis, avec des acides appropriés, en sels d'addition physiologiquement acceptables.

**9.** Composés de formule I et sels d'addition avec des acides physiologiquement acceptables de ces composés, comportant l'élément structural de formule II en position(s) $R^1$ et/ou $R^3$, selon une ou plusieurs des revendications 1 à 4, ou tels qu'obtenus conformément au procédé selon les revendications 5 à 8, pour utilisation en tant que médicament.

**10.** Médicament, caractérisé par une teneur en - ou consistant en - au moins un composé de formule I et/ou éventuellement au moins l'un de ses sels d'addition avec des acides physiologiquement acceptables, selon une ou plusieurs des revendications 1 à 4, ou en au moins un composé préparé conformément au procédé selon les revendications 5 à 8.

**11.** Médicament selon la revendication 10, pour la prévention et le traitement de maladies dans lesquelles est indiquée l'utilisation thérapeutique d'anti-inflammatoires, immunomodulateurs, agents désactivateurs de radicaux oxygénés et/ou inhibiteurs de la dégradation de l'acide arachidonique par la 5-lipo-oxygénase.

**12.** Médicament selon la revendication 10 ou 11, pour la prévention et le traitement de maladies inflammatoires, en particulier de maladies rhumatismales inflammatoires.

**13.** Procédé pour la préparation d'un médicament selon une ou plusieurs des revendications 10 à 12, caractérisé en ce que l'on met sous une forme pharmaceutique appropriée au moins un composé de formule I et/ou au moins un sel d'addition avec un acide physiologiquement acceptable d'un tel composé, comportant l'élément structural de formule II en position(s) $R^1$ et/ou $R^3$ et/ou au moins l'un des composés obtenus conformément au procédé selon les revendications 5 à 8, avec un véhicule physiologiquement acceptable et éventuellement d'autres adjuvants et/ou additifs.

**14.** Utilisation de composés de formule I et/ou éventuellement de leurs sels d'addition avec des acides physiologiquement acceptables, pour la fabrication de médicaments selon les revendications 11 et 12.

**15.** 2H-1,2,4-triazine-5-ones S-alkylées de formule V

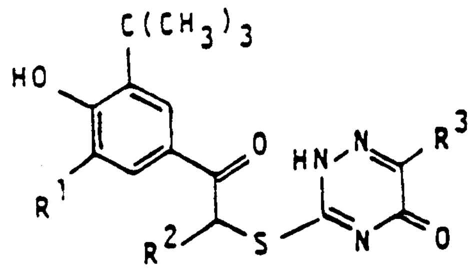

(V)

dans laquelle $R^1$ représente un groupe alkyle en $C_1$-$C_4$ et $R^2$ et $R^3$ ont les mêmes significations que dans la formule I selon la revendication 1.

**16.** Procédé pour la préparation de 2H-1,2,4-triazine-5-ones S-alkylées de formule V selon la revendication 12, caractérisé en ce que l'on met en réaction, dans des conditions basiques, des 3-mercapto-2H-1,2,4-triazine-5-ones de formule III

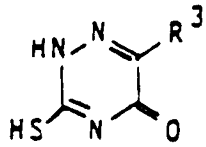

(III)

dans laquelle $R^3$ a la même signification que dans la formule I, avec des 2-halogéno-1-phénylalcanones de formule IV

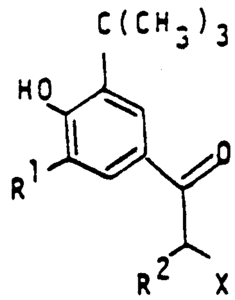

(IV)

dans laquelle $R^1$ représente un groupe alkyle en $C_1$-$C_4$ et $R^2$ a la même signification que dans la formule I, et X est un atome d'halogène.

**Revendications pour les Etats contractants suivants : ES, GR**

**1.** Procédé pour la préparation de 3-phényl-7H-thiazolo[3,2-b][1,2,4]triazine-7-ones substituées de formule générale I

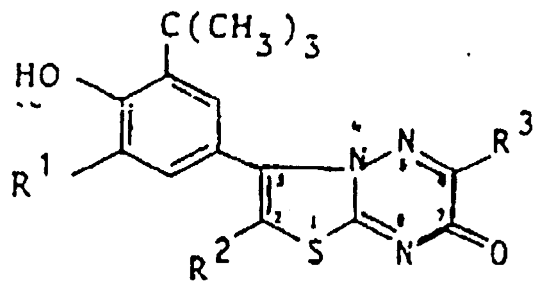

(I)

dans laquelle

$R^1$ représente un groupe alkyle à chaîne droite ou ramifiée ayant de 1 à 4 atomes de carbone, le groupe hydroxyméthyle ou un groupe aminométhyle de formule II

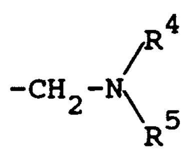

(II),

$R^2$ représente un atome d'hydrogène ou un radical alkyle ayant de 1 à 3 atomes de carbone, et

$R^3$ représente un atome d'hydrogène ou un groupe alkyle à chaîne droite ou ramifiée ayant de 1 à 4 atomes de carbone, le groupe hydroxyméthyle ou un groupe aminométhyle de formule II,

$R^4$ et $R^5$ étant identiques ou différents et représentant un atome d'hydrogène ou un radical alkyle à chaîne droite ou ramifiée ayant de 1 à 4 atomes de carbone, ou les deux radicaux, conjointement avec l'atome d'azote auxquels ils sont liés, formant un cycle saturé à 5-7 chaînons ayant de 4 à 6 atomes de carbone ou ayant 4 ou 5 atomes de carbone et en outre un autre hétéroatome sous forme de O, S ou $NR^6$, et $R^6$ représentant un atome d'hydrogène ou un groupe alkyle en $C_1$-$C_4$,

et des sels d'addition avec des acides physiologiquement acceptables des composés comportant l'élément structural de formule II en position(s) de $R^1$ et/ou $R^3$,

caractérisé en ce que

a) on fait réagir une 3-mercapto-2H-1,2,4-triazine-5-one de formule III

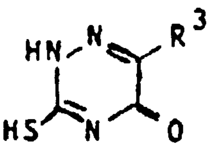

(III)

avec une 2-halogéno-1-phénylalcanone de formule IV

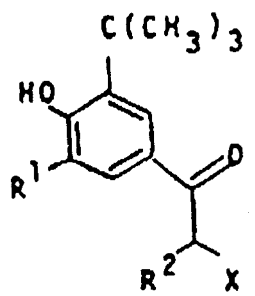

(IV)

pour aboutir à un composé de formule I, $R^1$ représentant le groupe alkyle en $C_1$-$C_4$ défini plus haut, et $R^2$ et $R^3$ ayant les significations données plus haut, et X représentant un atome d'halogène, ou

b) on fait réagir un composé de formule III avec un composé de formule IV (avec les significations des radicaux $R^1$, $R^2$ et X données pour celle-ci) dans des conditions basiques, pour aboutir d'abord à une 2H-1,2,4-triazine-5-one S-alkylée de formule V

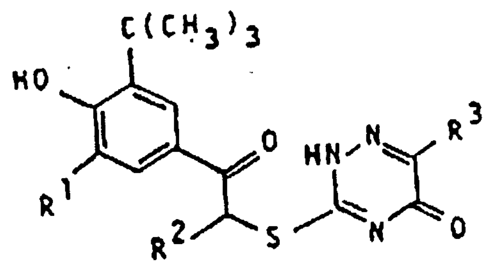

(V)

et on convertit celle-ci, par déshydratation, en un composé de formule I, $R^1$ représentant le groupe alkyle en $C_1$-$C_4$ défini plus haut, et $R^2$ et $R^3$ ayant les significations données à propos de la formule I, ou

c) on fait réagir une 2-halogéno-1-phénylalcanone de formule IV (avec la signification des radicaux $R^1$, $R^2$ et X données pour celle-ci), d'abord avec du thiosemicarbazide, pour aboutir à la 2-amino-6H-1,3,4-thiadiazine correspondante de formule VI

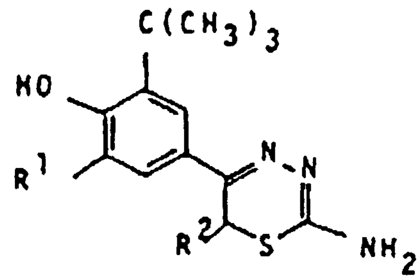

(VI)

et on convertit celle-ci par transposition, dans des conditions acides, en un 3-amino-2-imino-2,3-dihydrothiazole de formule VII

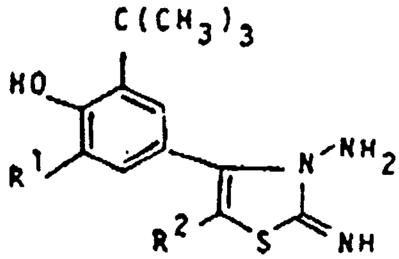

(VII)

qui est ensuite cyclocondensé avec un acide α-céto-carboxylique ou ses esters alkyliques de formule VIII

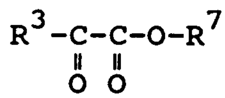

(VIII)

pour donner un composé de formule I, $R^1$ représentant le groupe alkyle en $C_1$-$C_4$ défini plus haut, $R^2$ ayant les significations données à propos de la formule I, $R^3$ représentant un atome d'hydrogène ou un groupe alkyle à chaîne droite ou ramifiée ayant de 1 à 4 atomes de carbone ou le groupe hydroxyméthyle, et $R^7$ représentant un atome d'hydrogène ou un radical alkyle ayant de 1 à 3 atomes de carbone, ou en ce que

d) à partir d'un composé de formule I qui porte en position(s) $R^1$ et/ou $R^3$ un radical hydroxyméthyle, d'abord on échange contre un halogène ou convertit en un ester d'acide sulfonique ou phosphorique actif son(ses) groupe(s) hydroxy et on le fait ensuite réagir avec une amine de formule IX

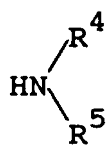

(IX)

pour aboutir à un composé de formule I comportant l'élément structural de formule II en position(s) de $R^1$ et/ou $R^3$, $R^2$, $R^4$, $R^5$ et éventuellement également $R^1$ ou $R^3$ ayant les significations données plus haut, et ce composé aminométhyle est soit isolé sous forme libre, soit converti, avec des acides appropriés, en sels d'addition physiologiquement acceptables, ou en ce que

e) pour la préparation d'un composé de formule I ayant la caractéristique de structure de formule II ou comportant le radical hydrométhyle en position de $R^1$, on part d'un composé de formule I dans lequel $R^1$ représente un atome d'hydrogène et, pour l'introduction du radical hydroxyméthyle, on le fait réagir avec du formaldéhyde, pour l'introduction de groupes aminométhyle de formule II substitués à l'azote, on le fait réagir avec les amines correspondantes de formule IX, en présence de formaldéhyde, ou pour l'introduction du radical aminométhyle non substitué, on le condense d'abord, dans des conditions acides, avec des N-hydroxyméthylacétamides de formule X

$R^8$-CO-NH-$CH_2$-OH (X)

dans laquelle $R^8$ représente le groupe trifluorométhyle, trichlorométhyle ou chlorométhyle, et ensuite on élimine par hydrolyse le radical acyle $R^8$-CO et $R^2$ à $R^5$ ayant les significations données plus haut, et les composés aminométhyle ainsi obtenus sont soit isolés sous forme libre, soit convertis, avec des acides appropriés, en sels d'addition physiologiquement acceptables.

**2.** Procédé selon la revendication 1, caractérisé en ce que l'on prépare des composés de formule I ayant au moins l'une des caractéristiques que $R^1$ représente le radical tert-butyle ou un groupe aminométhyle de formule II et que $R^2$ et $R^3$, indépendamment l'un de l'autre, représentent un atome d'hydrogène ou le groupe méthyle.

**3.** Procédé selon la revendication 1 ou 2, caractérisé en ce que l'on prépare des composés de formule I dans lesquels $R^1$ représente le groupe tert-butyle et en même temps $R^2$ et $R^3$ représentent, indépendamment l'un de l'autre, un atome d'hydrogène ou le groupe méthyle.

**4.** Procédé selon la revendication 3, caractérisé en ce que l'on prépare le composé de formule I dans lequel $R^1$ représente le groupe tert-butyle et $R^2$ et $R^3$ représentent des atomes d'hydrogène (= 3-(3,5-di-tert-butyl-4-hydroxyphényl)-7H-thiazolo[3,2-b][1,2,4]triazine-7-one).

**5.** Procédé pour la préparation des composés de formule I et des sels d'addition avec des acides physiologiquement acceptables de ces composés, comportant l'élément structural de formule II en position(s) $R^1$ et/ou $R^3$, selon une ou plusieurs des revendications 1 à 4, pour utilisation en tant que médicament.

**6.** Procédé pour la fabrication d'un médicament, caractérisé en ce que l'on met sous une forme pharmaceutique appropriée au moins un composé de formule I selon la définition donnée dans la revendication 1 et/ou au moins un sel d'addition avec un acide physiologiquement acceptable d'un tel composé, comportant l'élément structural de formule II en position(s) $R^1$ et/ou $R^3$ et/ou au moins l'un des composés obtenus conformément au procédé selon une ou plusieurs des revendications 1 à 4, avec un véhicule physiologiquement acceptable et éventuellement d'autres additifs et/ou adjuvants.

**7.** Utilisation de composés de formule I selon la définition donnée dans la revendication 1, ou tels qu'obtenus selon une ou plusieurs des revendications 1 à 4, et/ou éventuellement de leurs sels d'addition avec des acides physiologiquement acceptables, pour la fabrication de médicaments pour la prévention et le traitement de maladies dans lesquelles est indiquée l'utilisation thérapeutique d'antiinflammatoires, d'immunomodulateurs, agents désactivateurs de radicaux oxygénés et/ou inhibiteurs de la dégradation de l'acide arachidonique médiée par la 5-lipo-oxygénase, ou pour la prévention et le traitement de maladies inflammatoires, en particulier de maladies rhumatismales inflammatoires.

**8.** Composés de formule I et sels d'addition avec des acides physiologiquement acceptables des composés de formule I, comportant l'élément structural de formule II en position(s) $R^1$ et/ou $R^3$, préparés conformément au procédé selon une ou plusieurs des revendications 1 à 4.

**9.** Médicament préparé conformément au procédé selon la revendication 6.

**10.** 2H-1,2,4-triazine-5-ones S-alkylées de formule V

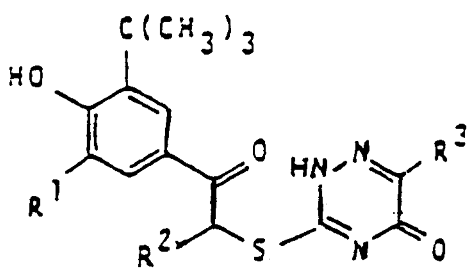

(V)

dans laquelle $R^1$ représente un groupe alkyle en $C_1$-$C_4$ et $R^2$ et $R^3$ ont les mêmes significations que dans la formule I selon la revendication 1.

**11.** Procédé pour la préparation de 2H-1,2,4-triazine-5-ones S-alkylées de formule V selon la revendication 12, caractérisé en ce que l'on met en réaction, dans des conditions basiques, des 3-mercapto-2H-1,2,4-triazine-5-ones de formule III

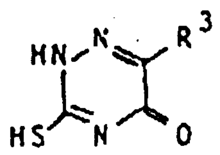

(III)

dans laquelle $R^3$ a la même signification que dans la formule I, avec des 2-halogéno-1-phénylalcanones de formule IV

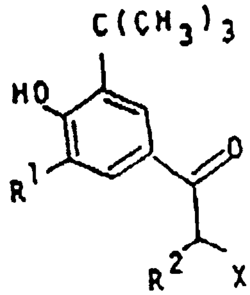

(IV)

dans laquelle $R^1$ représente un groupe alkyle en $C_1$-$C_4$ et $R^2$ a la même signification que dans la formule I, et X est un atome d'halogène.